(19) Europäisches Patentamt
European Patent Office
Office européen des brevets

(11) EP 3 437 656 A1

(12) # EUROPEAN PATENT APPLICATION
published in accordance with Art. 153(4) EPC

(43) Date of publication:
**06.02.2019 Bulletin 2019/06**

(21) Application number: **17774871.2**

(22) Date of filing: **27.03.2017**

(51) Int Cl.:
***A61K 39/395*** *(2006.01)* ***A61K 45/00*** *(2006.01)*
***A61P 35/00*** *(2006.01)* ***A61P 35/02*** *(2006.01)*
***C07K 16/28*** *(2006.01)* ***C07K 16/46*** *(2006.01)*
***C12N 15/09*** *(2006.01)* ***C12P 21/08*** *(2006.01)*

(86) International application number:
**PCT/JP2017/012263**

(87) International publication number:
**WO 2017/170334 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.03.2016 JP 2016064033**

(71) Applicant: **Toray Industries, Inc.**
**Tokyo 103-8666 (JP)**

(72) Inventors:
- **FUJITA, Takayuki**
  **Kamakura-shi**
  **Kanagawa 248-8555 (JP)**
- **OKANO, Fumiyoshi**
  **Kamakura-shi**
  **Kanagawa 248-8555 (JP)**

(74) Representative: **Mewburn Ellis LLP**
**City Tower**
**40 Basinghall Street**
**London EC2V 5DE (GB)**

(54) **PHARMACEUTICAL COMPOSITION FOR TREATING AND/OR PREVENTING CANCER**

(57) The present invention relates to a pharmaceutical composition for treatment and/or prevention of a cancer, which comprises, as an active ingredient, an antibody or fragment thereof having an immunological reactivity with an MRAP2 protein having the amino acid sequence shown in SEQ ID NO: 2, 4, 6, or 8 or an amino acid sequence having 80% or more sequence identity with the amino acid sequence, or with a fragment of the MRAP2 protein comprising 7 or more consecutive amino acids.

EP 3 437 656 A1

SEQUENCE LISTING

<110> TORAY INDUSTRIES, INC.

<120> Pharmaceutical composition for treating and/or preventing a cancer

<130> PH-6862-PCT

<150> JP 2016-064033
<151> 2016-03-28

<160> 42

<170> PatentIn version 3.5

<210> 1
<211> 2229
<212> DNA
<213> Homo sapiens

<220>
<221> CDS
<222> (191)..(808)

<400> 1
```
tccggcgccc cgcgccgcct ccgctgcggg tcgggagcgc gcgtctccgc cgcacctcgg      60

atctaggagc tactcgcccg gccctgggcg gtgggaggcg gcggcggcgg cggcgctcgc     120

gcacctcgga ggagccagga gccggaacca gggccgagcc cgcgggccgg ggctagccag     180

ccggtcggag atg tcc gcc cag agg tta att tct aac aga acc tcc cag        229
           Met Ser Ala Gln Arg Leu Ile Ser Asn Arg Thr Ser Gln
           1               5                   10

caa tcg gca tct aat tct gat tac acc tgg gaa tat gaa tat tat gag       277
Gln Ser Ala Ser Asn Ser Asp Tyr Thr Trp Glu Tyr Glu Tyr Tyr Glu
    15                  20                  25

att gga cca gtt tcc ttt gaa gga ctg aag gct cat aaa tat tcc att       325
Ile Gly Pro Val Ser Phe Glu Gly Leu Lys Ala His Lys Tyr Ser Ile
30                  35                  40                  45

gtg att gga ttt tgg gtt ggt ctt gca gtc ttc gtg att ttt atg ttt       373
Val Ile Gly Phe Trp Val Gly Leu Ala Val Phe Val Ile Phe Met Phe
                50                  55                  60

ttt gtg ctg acc ttg ctg acc aag aca gga gcc cca cac caa gac aat       421
Phe Val Leu Thr Leu Leu Thr Lys Thr Gly Ala Pro His Gln Asp Asn
            65                  70                  75

gca gag tcc tca gag aag aga ttc aga atg aac agc ttt gtg tca gac       469
Ala Glu Ser Ser Glu Lys Arg Phe Arg Met Asn Ser Phe Val Ser Asp
        80                  85                  90

ttt gga aga cct ctg gag cca gat aaa gta ttt tct cgc caa ggc aac       517
Phe Gly Arg Pro Leu Glu Pro Asp Lys Val Phe Ser Arg Gln Gly Asn
    95                  100                 105

gag gag tcc agg tct ctc ttt cac tgc tac atc aat gag gtg gaa cgc       565
Glu Glu Ser Arg Ser Leu Phe His Cys Tyr Ile Asn Glu Val Glu Arg
```

```
110                 115                 120                 125

ttg gac aga gcc aaa gct tgt cac cag acc aca gcc ctt gac agt gac        613
Leu Asp Arg Ala Lys Ala Cys His Gln Thr Thr Ala Leu Asp Ser Asp
                130                 135                 140

gtc caa ctc cag gaa gcc atc aga agc agt ggg cag cca gag gag gag        661
Val Gln Leu Gln Glu Ala Ile Arg Ser Ser Gly Gln Pro Glu Glu Glu
            145                 150                 155

ctg aac agg ctc atg aag ttt gac atc ccc aac ttt gtg aac aca gac        709
Leu Asn Arg Leu Met Lys Phe Asp Ile Pro Asn Phe Val Asn Thr Asp
        160                 165                 170

cag aac tac ttt ggg gag gat gat ctt ctg att tct gaa cca cct att        757
Gln Asn Tyr Phe Gly Glu Asp Asp Leu Leu Ile Ser Glu Pro Pro Ile
    175                 180                 185

gtt ctg gaa act aag cca ctt tcc cag acc tca cac aaa gac ctg gat        805
Val Leu Glu Thr Lys Pro Leu Ser Gln Thr Ser His Lys Asp Leu Asp
190                 195                 200                 205

tga gaaacatgct ctgtaaaggg tcttcctgaa gatgtggatt ctatctttat             858

gtagcaagaa atctacatcc accaaaattg tgtgtgtttg ggggagagag agacatagag      918

atagagacag agaggcagag aagagacccc tttagaagag agctgagctg attaagctga      978

gtggtttttt gttttgtttt gtttttgctt tttaatacat ttggagcttt gggagtatta     1038

aagtatttac accaagcttg tccaacccgt ggcatgtgtc ccaggacagc tttgaatgtg     1098

gcccaataca aattttaaac tttattaaaa catgagtttt gttttttttt ttgctatttt     1158

ttttaaagct cgtcagctat cgttagtgtt agtgtacttt atgtgtggcc caagacaact     1218

cttcttccag tgtggcacag ggaagctaaa agattggaca cctctgattt atactagctc     1278

gttttgcttg ttgaaaaatt tggccaaata cctattgtca gcattcttgg gtgaggatta     1338

gcctaccatg ttctaatctg gccctgccac tactatgctc tacctttggt gagttgcttt     1398

acctctctgg gctgccccat ttttaactgt aggttgacag gtctagagtg atccatccca     1458

cctctaatat tttgtgaatt tatgactttg ccttcagatg aggctgagct atacataaaa     1518

cagtataaac tagggtactg cctcgtatct cttgtaggct ctctcaaatc tctgtacctt     1578

ccacttaacc ctaattgagc caagctttag tcaggggatc tggttgtcta ccagaatgtc     1638

aggagactca tcttacacag tcatggtggc caatgtttct ggtgggttgt gctgaaacag     1698

ctcttctgag aacttccaac cacccatgct ctaacctgga gacagccatc ccctgcctca     1758

gaataagtac caattcgtag tacatgtatg gtactcttgt ccccaagaaa tgttaggaag     1818

cttgtcagct gaatgagagg aggtgccttc tgggtatctc tgtgttggtg tatctgtgcc     1878

attggctaca gaacaagaaa aatactattt gccatgctat taccttggca gatgtgtagg     1938

tgatagtcat ctggctttga gctgagatgg tcagtgggtt gtaaattccc cactagcaga     1998

tattcagggt ggcctgagtt atgtaaacaa gtgagcaaca cagctttaat ttcatggagg     2058
```

```
aatcaaagct gcacactggt attaaaacaa cttgattttg cgcacacagt tgcatgcatg     2118

gcaagctgtt aacctctggg tggcattttc attatgaatt tgttcaccac ctgtcttgct     2178

taagctacaa aataaatgca tttgactgca cagaaaaaaa aaaaaaaaaa a              2229


<210>  2
<211>  205
<212>  PRT
<213>  Homo sapiens

<400>  2

Met Ser Ala Gln Arg Leu Ile Ser Asn Arg Thr Ser Gln Gln Ser Ala
1               5                   10                  15


Ser Asn Ser Asp Tyr Thr Trp Glu Tyr Glu Tyr Tyr Glu Ile Gly Pro
            20                  25                  30


Val Ser Phe Glu Gly Leu Lys Ala His Lys Tyr Ser Ile Val Ile Gly
        35                  40                  45


Phe Trp Val Gly Leu Ala Val Phe Val Ile Phe Met Phe Phe Val Leu
    50                  55                  60


Thr Leu Leu Thr Lys Thr Gly Ala Pro His Gln Asp Asn Ala Glu Ser
65                  70                  75                  80


Ser Glu Lys Arg Phe Arg Met Asn Ser Phe Val Ser Asp Phe Gly Arg
                85                  90                  95


Pro Leu Glu Pro Asp Lys Val Phe Ser Arg Gln Gly Asn Glu Glu Ser
            100                 105                 110


Arg Ser Leu Phe His Cys Tyr Ile Asn Glu Val Glu Arg Leu Asp Arg
        115                 120                 125


Ala Lys Ala Cys His Gln Thr Thr Ala Leu Asp Ser Asp Val Gln Leu
    130                 135                 140


Gln Glu Ala Ile Arg Ser Ser Gly Gln Pro Glu Glu Glu Leu Asn Arg
145                 150                 155                 160


Leu Met Lys Phe Asp Ile Pro Asn Phe Val Asn Thr Asp Gln Asn Tyr
                165                 170                 175


Phe Gly Glu Asp Asp Leu Leu Ile Ser Glu Pro Pro Ile Val Leu Glu
            180                 185                 190
```

```
Thr Lys Pro Leu Ser Gln Thr Ser His Lys Asp Leu Asp
        195                 200                 205


<210>  3
<211>  1967
<212>  DNA
<213>  Canis familiaris


<220>
<221>  CDS
<222>  (175)..(795)

<400>  3
tatgtaagcc attttagtca gctgtcatgt gaatgcaggg gacagaggcg atgtaatgcg      60

cacccacgta agagtatctg cttgctatga agatgtggat gtcagactgc taactttctt     120

gcagaggacc actgttactg cgaggttatc tcttcctcac ttagaagggt ggag atg       177
                                                            Met
                                                            1

tct gcc cag aga tta att tct aac aga aca tcc cag cca tct gca cct       225
Ser Ala Gln Arg Leu Ile Ser Asn Arg Thr Ser Gln Pro Ser Ala Pro
            5                   10                  15

aat tct gat tac acc tgg gaa tat gaa tat tat gaa att gga cca gtg       273
Asn Ser Asp Tyr Thr Trp Glu Tyr Glu Tyr Tyr Glu Ile Gly Pro Val
        20                  25                  30

tcc ttt gaa gga ctg aag gct cat aaa tat tcc att gtg att gga ttt       321
Ser Phe Glu Gly Leu Lys Ala His Lys Tyr Ser Ile Val Ile Gly Phe
    35                  40                  45

tgg gtt ggt ctc gct gtc ttt gtg att ttc atg ttt ttt gtg ctg act       369
Trp Val Gly Leu Ala Val Phe Val Ile Phe Met Phe Phe Val Leu Thr
50                  55                  60                  65

ttg ctg acc aag acg gga gct cca cac caa gac aat gca gaa tct tca       417
Leu Leu Thr Lys Thr Gly Ala Pro His Gln Asp Asn Ala Glu Ser Ser
                70                  75                  80

gag aag aga ttt aga atg aat agc ttt gtg tca gac ttt gga aga cca       465
Glu Lys Arg Phe Arg Met Asn Ser Phe Val Ser Asp Phe Gly Arg Pro
            85                  90                  95

ctg gag cca gat aag gtg ttt tct cga cag ggc aat gat gaa tcc agg       513
Leu Glu Pro Asp Lys Val Phe Ser Arg Gln Gly Asn Asp Glu Ser Arg
        100                 105                 110

tct ctc ttt cat tgc tac atc aat gaa gtg gaa cac ttg gat agg gct       561
Ser Leu Phe His Cys Tyr Ile Asn Glu Val Glu His Leu Asp Arg Ala
    115                 120                 125

aaa gtt tgt cat cag acc acg gtc ctt gac agc agt gtt cga ctc cag       609
Lys Val Cys His Gln Thr Thr Val Leu Asp Ser Ser Val Arg Leu Gln
130                 135                 140                 145

gaa gcc att aga agc aat ggg cgt cca gag gag gag ctg aat agg ctt       657
Glu Ala Ile Arg Ser Asn Gly Arg Pro Glu Glu Glu Leu Asn Arg Leu
                150                 155                 160
```

atg aag ttt gat atc cct aac ttt gtg aat aca gac cag aac tcc tcc 705
Met Lys Phe Asp Ile Pro Asn Phe Val Asn Thr Asp Gln Asn Ser Ser
165 170 175

ttt ggg gag gat gat ctt cta att tca gaa cca cct att gtt cta gaa 753
Phe Gly Glu Asp Asp Leu Leu Ile Ser Glu Pro Pro Ile Val Leu Glu
180 185 190

aat aag cca gtt tcc cag acc tca cac aaa gac ctg gat tga 795
Asn Lys Pro Val Ser Gln Thr Ser His Lys Asp Leu Asp
195 200 205

gaaactactc tgtaaagtgt cttcctgaag atgttgggtc tgtctttgta aagcaagaaa 855

tctccattca ccaagattgt gtgtatgtgt gtgtgggggg gtagctaagt gggggtgtta 915

tgagagatag aaacagagga gagagccccc tcaaaaacga gccgaataag ctgagtttct 975

atgcctttaa acacagttca ggctttttgg ggaataaggt acttatatgg tttcatcctc 1035

ttcgttgttg aaaaatttgg ccacatactc agtgtcaacg ttcttgaatg ggggttagca 1095

tactgtgttc cagtctggcc ctgccaattc catgctctac ctttagcaag ttgctttacc 1155

tctctgggct gccacattgt taaccgtaac atggggagtc tggagtagat tattcatcct 1215

agctctaata ttttgtgaac ttgtgacttt gcatcgagaa gaggctgggt tatatgtaaa 1275

tcaatataaa ctatggcact gcctcctatc tcttgtagga tcccccatgt cagtaccttc 1335

cactcaacct taactgagcc cagcttttgt caagggtcca agcatctaga ggatgtcaga 1395

cgactcacct cacagtcacc gtggccaaca tttctgttgg ttgtatgaaa atagctcttc 1455

tgagaacctc cagccaccca tggtaaaacg tagggacagc catgcccctc ctcttctaga 1515

atgagtacca atccatgata cattgcatgg cactcctatc cccagaaatg tagggaaggt 1575

tgtcagctga gtgatgacag gtgccttttg tgtctctgtt ggtgaacatg tgtctggtca 1635

catttctgtg tggttggcta ctgaacaaga aaagtgccat ttgccatgct ctttacttgg 1695

taggggtgta ggtgatggtc atgtggctcg tgtgtgggct ttgagctgag gtggtgaatg 1755

gattgtaaat tccccaccaa cagatgtgca gggtggcctg gtgcaacaca gttaatttcc 1815

cgaaggaatc tagtcctggt attgggaaaa acttggtctt gcatacacag ttgcatgtgc 1875

agccagctgc taatctctgg gtggcatttt cattatgaat ttgttcacca tctgtcttgc 1935

ttaagcaaaa aaaataaatg catttgattg ca 1967

<210> 4
<211> 206
<212> PRT
<213> Canis familiaris

<400> 4

Met Ser Ala Gln Arg Leu Ile Ser Asn Arg Thr Ser Gln Pro Ser Ala
1 5 10 15

```
Pro Asn Ser Asp Tyr Thr Trp Glu Tyr Glu Tyr Tyr Glu Ile Gly Pro
            20                  25                  30

Val Ser Phe Glu Gly Leu Lys Ala His Lys Tyr Ser Ile Val Ile Gly
        35                  40                  45

Phe Trp Val Gly Leu Ala Val Phe Val Ile Phe Met Phe Phe Val Leu
    50                  55                  60

Thr Leu Leu Thr Lys Thr Gly Ala Pro His Gln Asp Asn Ala Glu Ser
65                  70                  75                  80

Ser Glu Lys Arg Phe Arg Met Asn Ser Phe Val Ser Asp Phe Gly Arg
                85                  90                  95

Pro Leu Glu Pro Asp Lys Val Phe Ser Arg Gln Gly Asn Asp Glu Ser
            100                 105                 110

Arg Ser Leu Phe His Cys Tyr Ile Asn Glu Val Glu His Leu Asp Arg
        115                 120                 125

Ala Lys Val Cys His Gln Thr Thr Val Leu Asp Ser Ser Val Arg Leu
    130                 135                 140

Gln Glu Ala Ile Arg Ser Asn Gly Arg Pro Glu Glu Glu Leu Asn Arg
145                 150                 155                 160

Leu Met Lys Phe Asp Ile Pro Asn Phe Val Asn Thr Asp Gln Asn Ser
                165                 170                 175

Ser Phe Gly Glu Asp Asp Leu Leu Ile Ser Glu Pro Pro Ile Val Leu
            180                 185                 190

Glu Asn Lys Pro Val Ser Gln Thr Ser His Lys Asp Leu Asp
        195                 200                 205
```

<210> 5
<211> 1849
<212> DNA
<213> Felis catus

<220>
<221> CDS
<222> (72)..(692)

<400> 5

```
gaggcggtgg cgaagctcac gcacctcgga ggagcggtgc ccgcgggccg gggagcgcca      60

gccgggtaga g atg tct gcc cag agg tta att tct aac aga aca tcc cag      110
```

```
             Met Ser Ala Gln Arg Leu Ile Ser Asn Arg Thr Ser Gln
             1               5                   10

caa tct gca tct aat tct gat tac acc tgg gaa tat gaa tat tat gag        158
Gln Ser Ala Ser Asn Ser Asp Tyr Thr Trp Glu Tyr Glu Tyr Tyr Glu
    15                  20                  25

att gga cca gtt tcc ttt gaa gga ctg aag gct cat aaa tat tcc att        206
Ile Gly Pro Val Ser Phe Glu Gly Leu Lys Ala His Lys Tyr Ser Ile
30                  35                  40                  45

gtg att gga ttt tgg gtt ggt ctt gct gtc ttc gtg att ttc atg ttt        254
Val Ile Gly Phe Trp Val Gly Leu Ala Val Phe Val Ile Phe Met Phe
                50                  55                  60

ttt gtg ctg act ttg ctg acc aag acg gga gct cca cac caa gac aat        302
Phe Val Leu Thr Leu Leu Thr Lys Thr Gly Ala Pro His Gln Asp Asn
            65                  70                  75

gca gag tct tca gag aag aga ttc aga atg aat agc ttt gtg tca gac        350
Ala Glu Ser Ser Glu Lys Arg Phe Arg Met Asn Ser Phe Val Ser Asp
        80                  85                  90

ttc gga aga cca ctg gag cca gat aag gtg ttt tct cga cag ggc aat        398
Phe Gly Arg Pro Leu Glu Pro Asp Lys Val Phe Ser Arg Gln Gly Asn
    95                  100                 105

gag gaa tcc agg tct ctc ttt cac tgc tac atc aac gaa gtg gaa cac        446
Glu Glu Ser Arg Ser Leu Phe His Cys Tyr Ile Asn Glu Val Glu His
110                 115                 120                 125

ttg gat agg gct aaa gct tgt cag cag acc aca gcc ctt gac agc tgt        494
Leu Asp Arg Ala Lys Ala Cys Gln Gln Thr Thr Ala Leu Asp Ser Cys
                130                 135                 140

gtt caa ctg cag gaa gcc att aga agc aac ggg cgg cca gag gag gag        542
Val Gln Leu Gln Glu Ala Ile Arg Ser Asn Gly Arg Pro Glu Glu Glu
            145                 150                 155

ttg aac agg ctc atg aag ttt gac atc ccc aac ttc gtg aac aca gac        590
Leu Asn Arg Leu Met Lys Phe Asp Ile Pro Asn Phe Val Asn Thr Asp
        160                 165                 170

cag aac tcc tcc ttt ggg gag gat gat ctt ctg att tca gaa cca cct        638
Gln Asn Ser Ser Phe Gly Glu Asp Asp Leu Leu Ile Ser Glu Pro Pro
    175                 180                 185

att gtt cta gaa aat aag cca gtt tcc cag acg tca cac aaa gac ctg        686
Ile Val Leu Glu Asn Lys Pro Val Ser Gln Thr Ser His Lys Asp Leu
190                 195                 200                 205

gat tga gaaacgtact ctgtaaagtg tcttcctgga gatgttggat ccgtctttgt         742
Asp

aaagcaagaa atctccactg accacagttg tttgtgtgtg ttggggggga gacatgggag      802

acagagatag aaagaaagac acagagaaga gagccccctc agaaaagagc tgaagaagct      862

gagtttctgt gcctttaaac acagttcagg ctttttgag aataaagtat ttgcatggtc       922

tcatctttct tgttgttgaa aagtttggct gcacagagtg tcagtgttct tgaatggggg      982
```

ttagcatgct gcattccagt ctggccctgc caccaccacg ctctattttt agcaagttgc 1042

tttacctctc tgggctgcca cattgttcat tgtaacatga ggagtttgaa gtagatgact 1102

catcccagct ctaatatttt gtgaatttgt gactttgcat ccagaagaag ctggaatgta 1162

cataatgcag tataaaccag ggcactgcct cctatctctt gtaggatgcc ctatgttggt 1222

accttccact cagcccttaa ctgagcccag cttttgtcag aggtccaagc atctatgagg 1282

aggtcagaag acacatctct cagtcaccat ggccaacatt tctttgattg tactgaaaaa 1342

gctcttctga gaacctccag ccacccatgc taaaagctag ggccagccct gcccctcttc 1402

tcccagaatg aaccaattca tggtaccaat tcatggtaca tcgcatggca ctcttgttcc 1462

cagaaatgta gagaaggttg tcagctcagt gagaggaggt gccttttgta tctctgtgtt 1522

ggcgtgcgtc cgtcacaaga agagtacttg ccatgctatt tacttggcag atgtgtaggt 1582

gatagtcatg tgactcgtgt atggactttg agctgagatg gtgaatgggt tgtaaatccc 1642

caccaacaga tatgcagggt ggcctggtgc aacacagagt taatttcatg aaagaatcca 1702

gtctgcacac tggtattgga aacaacttgg ttttgcgcac acagttgctt gcatggccaa 1762

ctgctaatct ctgggtggca ttttcatcat gagtttgttt accacctgtc ttgctaaagc 1822

taaaaaataa atgcatttgc acaggaa 1849

<210> 6
<211> 206
<212> PRT
<213> Felis catus

<400> 6

Met Ser Ala Gln Arg Leu Ile Ser Asn Arg Thr Ser Gln Gln Ser Ala
1 5 10 15

Ser Asn Ser Asp Tyr Thr Trp Glu Tyr Glu Tyr Tyr Glu Ile Gly Pro
20 25 30

Val Ser Phe Glu Gly Leu Lys Ala His Lys Tyr Ser Ile Val Ile Gly
35 40 45

Phe Trp Val Gly Leu Ala Val Phe Val Ile Phe Met Phe Phe Val Leu
50 55 60

Thr Leu Leu Thr Lys Thr Gly Ala Pro His Gln Asp Asn Ala Glu Ser
65 70 75 80

Ser Glu Lys Arg Phe Arg Met Asn Ser Phe Val Ser Asp Phe Gly Arg
85 90 95

Pro Leu Glu Pro Asp Lys Val Phe Ser Arg Gln Gly Asn Glu Glu Ser

100 105 110

Arg Ser Leu Phe His Cys Tyr Ile Asn Glu Val Glu His Leu Asp Arg
115 120 125

Ala Lys Ala Cys Gln Gln Thr Thr Ala Leu Asp Ser Cys Val Gln Leu
130 135 140

Gln Glu Ala Ile Arg Ser Asn Gly Arg Pro Glu Glu Glu Leu Asn Arg
145 150 155 160

Leu Met Lys Phe Asp Ile Pro Asn Phe Val Asn Thr Asp Gln Asn Ser
165 170 175

Ser Phe Gly Glu Asp Asp Leu Leu Ile Ser Glu Pro Pro Ile Val Leu
180 185 190

Glu Asn Lys Pro Val Ser Gln Thr Ser His Lys Asp Leu Asp
195 200 205

<210> 7
<211> 1948
<212> DNA
<213> Mus musculus

<220>
<221> CDS
<222> (94)..(717)

<400> 7
gttcactggc ggcggcggca cggcggcggt gaggctcgag caccggagga gctgaaccgt 60

gtaaagcctg cggtaaccca ggacaggacg ggg atg gag atg tct gcc cag agg 114
Met Glu Met Ser Ala Gln Arg
1 5

ctg gct tct aac agg aca tcc cca cag tca cca tcg aac tct gac tac 162
Leu Ala Ser Asn Arg Thr Ser Pro Gln Ser Pro Ser Asn Ser Asp Tyr
10 15 20

acc tgg gaa tac gag tac tat gag atc gga cca gtt tcc ttt gaa gga 210
Thr Trp Glu Tyr Glu Tyr Tyr Glu Ile Gly Pro Val Ser Phe Glu Gly
25 30 35

ctg aag gct cat aaa tat tcc att gtg att gga ttc tgg gtt ggt ctt 258
Leu Lys Ala His Lys Tyr Ser Ile Val Ile Gly Phe Trp Val Gly Leu
40 45 50 55

gcg gtc ttt gtg att ttc atg ttt ttt gtg ctg act ttg ctg acg aag 306
Ala Val Phe Val Ile Phe Met Phe Phe Val Leu Thr Leu Leu Thr Lys
60 65 70

aca ggt gcc cca cac caa gac aac gcg gag tcc tca gag agg agg ttc 354
Thr Gly Ala Pro His Gln Asp Asn Ala Glu Ser Ser Glu Arg Arg Phe
75 80 85

```
cgc atg aac agc ttt gtg tca gac ttc ggg aag ccg ctg gag tca gac       402
Arg Met Asn Ser Phe Val Ser Asp Phe Gly Lys Pro Leu Glu Ser Asp
        90                  95                  100

aag gtg ttt tct cgt cag ggc aac gag gag tcc agg tct ctg ttc cac       450
Lys Val Phe Ser Arg Gln Gly Asn Glu Glu Ser Arg Ser Leu Phe His
    105                 110                 115

tgt tac atc aat gaa gta gaa cac ttg gac agg gtt aaa gtt tgc cac       498
Cys Tyr Ile Asn Glu Val Glu His Leu Asp Arg Val Lys Val Cys His
120                 125                 130                 135

caa aca aca gcc atc gac agt gat gtc cac ctc cag gaa gcc agc aga       546
Gln Thr Thr Ala Ile Asp Ser Asp Val His Leu Gln Glu Ala Ser Arg
                140                 145                 150

agc agt ggg agg cct gag gag gag cta gcc agg ttc atg aag ttt gac       594
Ser Ser Gly Arg Pro Glu Glu Glu Leu Ala Arg Phe Met Lys Phe Asp
            155                 160                 165

atc ccc aac ttt gtg aac aca gag cag agc tcc ttt ggg gag gat gat       642
Ile Pro Asn Phe Val Asn Thr Glu Gln Ser Ser Phe Gly Glu Asp Asp
        170                 175                 180

ctc ctg att tcg gaa gca ccc gtt ctt cta gaa aac aag cca gtt tcc       690
Leu Leu Ile Ser Glu Ala Pro Val Leu Leu Glu Asn Lys Pro Val Ser
    185                 190                 195

cag aca tca cgc ata gac ctg gac tga gagacacttg tgccttactg             737
Gln Thr Ser Arg Ile Asp Leu Asp
200                 205

aggatgtgtt ctgtctctgt acagcaagaa accgcaagct acccacactc gtgtgtgtgt      797

gtgtgtgtgt gtgtgtgtgt gtgtgtgtag agagagagag agagagagag agagagagag      857

agagagagag gaggtgaggg agggggagag gactaggcta ggactgggat gtgatagagg      917

ggcagaattt ggagatggaa ggcaccattt tggtttgggg gagatgatat ttttcttttt      977

tggattaaaa aaatgcattg acagtattaa ggcttcttta ttgaaacaat tagccacata     1037

tatgcaattg agccccctcc tgggtgaggg ttaggttact gcagtctgat ctagcccttt     1097

cccaccattc tctgcctgta acaagttgct ttacctctct ggactgccat ggttttacat     1157

gtaaggtggc ctgtctggag tagatgacct atcccagctc tagaattttt gtggctttca     1217

gacttagcgt ttaagacatg ctaagaaaca cataaacagt agataataga acatggcacc     1277

ctggctttca gggcaggttc tccagggctt ggtaccctcc ctttgctcag cccttcacag     1337

agtctcactt tagctaggga aacctttacc aggagcacag gagcctcaca tgcactgtgg     1397

acagtgtctc tgcgagtttt gccgcagcag tttatgtgat gaactccacc caccccggct     1457

ttaaactggg gcagctttga ctccagtgag taccagccta aactgtttca caagcatccg     1517

tgctctcaaa aactgctagg gcagattgtc atctgcctga tgtgggtccc ttctcggtgt     1577

ctccgagttt gcatcctgtg ctcgcgcttc tgaactcttt ggcttcagaa caagacgctc     1637
```

```
cttgccatgg tctgcgtggc agatgtgtgg gtgtcggctg cctggcttgg agctgagagg     1697

gtcagtgggt tgtttgtgcc acagtagcag atgttcagga ttgcgatgat aatttgagca     1757

acatgatgtt catttcgtgg aggaagcaac cctttgctgg tgtcggaaag aactcagcct     1817

tgcacacaga gagttgtatg catgagcagc tgctaatcga tgggtggcat ctactttacg     1877

aatttgttca ccctttgtct tgcttaagtt gcataataaa tgcatttgat cgcaaaaaaa     1937

aaaaaaaaaa a                                                          1948


<210>  8
<211>  207
<212>  PRT
<213>  Mus musculus

<400>  8

Met Glu Met Ser Ala Gln Arg Leu Ala Ser Asn Arg Thr Ser Pro Gln
1               5                   10                  15


Ser Pro Ser Asn Ser Asp Tyr Thr Trp Glu Tyr Glu Tyr Tyr Glu Ile
            20                  25                  30


Gly Pro Val Ser Phe Glu Gly Leu Lys Ala His Lys Tyr Ser Ile Val
        35                  40                  45


Ile Gly Phe Trp Val Gly Leu Ala Val Phe Val Ile Phe Met Phe Phe
    50                  55                  60


Val Leu Thr Leu Leu Thr Lys Thr Gly Ala Pro His Gln Asp Asn Ala
65                  70                  75                  80


Glu Ser Ser Glu Arg Arg Phe Arg Met Asn Ser Phe Val Ser Asp Phe
                85                  90                  95


Gly Lys Pro Leu Glu Ser Asp Lys Val Phe Ser Arg Gln Gly Asn Glu
            100                 105                 110


Glu Ser Arg Ser Leu Phe His Cys Tyr Ile Asn Glu Val Glu His Leu
        115                 120                 125


Asp Arg Val Lys Val Cys His Gln Thr Thr Ala Ile Asp Ser Asp Val
    130                 135                 140


His Leu Gln Glu Ala Ser Arg Ser Ser Gly Arg Pro Glu Glu Glu Leu
145                 150                 155                 160


Ala Arg Phe Met Lys Phe Asp Ile Pro Asn Phe Val Asn Thr Glu Gln
                165                 170                 175
```

```
Ser Ser Phe Gly Glu Asp Asp Leu Leu Ile Ser Glu Ala Pro Val Leu
            180                 185                 190

Leu Glu Asn Lys Pro Val Ser Gln Thr Ser Arg Ile Asp Leu Asp
        195                 200                 205
```

<210> 9
<211> 132
<212> DNA
<213> Homo sapiens

<220>
<221> CDS
<222> (1)..(132)

<400> 9

```
atg tcc gcc cag agg tta att tct aac aga acc tcc cag caa tcg gca          48
Met Ser Ala Gln Arg Leu Ile Ser Asn Arg Thr Ser Gln Gln Ser Ala
1               5                   10                  15

tct aat tct gat tac acc tgg gaa tat gaa tat tat gag att gga cca          96
Ser Asn Ser Asp Tyr Thr Trp Glu Tyr Glu Tyr Tyr Glu Ile Gly Pro
            20                  25                  30

gtt tcc ttt gaa gga ctg aag gct cat aaa tat tcc                          132
Val Ser Phe Glu Gly Leu Lys Ala His Lys Tyr Ser
        35                  40
```

<210> 10
<211> 44
<212> PRT
<213> Homo sapiens

<400> 10

```
Met Ser Ala Gln Arg Leu Ile Ser Asn Arg Thr Ser Gln Gln Ser Ala
1               5                   10                  15

Ser Asn Ser Asp Tyr Thr Trp Glu Tyr Glu Tyr Tyr Glu Ile Gly Pro
            20                  25                  30

Val Ser Phe Glu Gly Leu Lys Ala His Lys Tyr Ser
        35                  40
```

<210> 11
<211> 417
<212> DNA
<213> Homo sapiens

<220>
<221> CDS
<222> (1)..(417)

<400> 11

```
acc aag aca gga gcc cca cac caa gac aat gca gag tcc tca gag aag          48
Thr Lys Thr Gly Ala Pro His Gln Asp Asn Ala Glu Ser Ser Glu Lys
1               5                   10                  15

aga ttc aga atg aac agc ttt gtg tca gac ttt gga aga cct ctg gag          96
Arg Phe Arg Met Asn Ser Phe Val Ser Asp Phe Gly Arg Pro Leu Glu
            20                  25                  30

cca gat aaa gta ttt tct cgc caa ggc aac gag gag tcc agg tct ctc         144
Pro Asp Lys Val Phe Ser Arg Gln Gly Asn Glu Glu Ser Arg Ser Leu
        35                  40                  45

ttt cac tgc tac atc aat gag gtg gaa cgc ttg gac aga gcc aaa gct         192
Phe His Cys Tyr Ile Asn Glu Val Glu Arg Leu Asp Arg Ala Lys Ala
    50                  55                  60

tgt cac cag acc aca gcc ctt gac agt gac gtc caa ctc cag gaa gcc         240
Cys His Gln Thr Thr Ala Leu Asp Ser Asp Val Gln Leu Gln Glu Ala
65                  70                  75                  80

atc aga agc agt ggg cag cca gag gag gag ctg aac agg ctc atg aag         288
Ile Arg Ser Ser Gly Gln Pro Glu Glu Glu Leu Asn Arg Leu Met Lys
                85                  90                  95

ttt gac atc ccc aac ttt gtg aac aca gac cag aac tac ttt ggg gag         336
Phe Asp Ile Pro Asn Phe Val Asn Thr Asp Gln Asn Tyr Phe Gly Glu
            100                 105                 110

gat gat ctt ctg att tct gaa cca cct att gtt ctg gaa act aag cca         384
Asp Asp Leu Leu Ile Ser Glu Pro Pro Ile Val Leu Glu Thr Lys Pro
        115                 120                 125

ctt tcc cag acc tca cac aaa gac ctg gat tga                             417
Leu Ser Gln Thr Ser His Lys Asp Leu Asp
    130                 135


<210>  12
<211>  138
<212>  PRT
<213>  Homo sapiens

<400>  12

Thr Lys Thr Gly Ala Pro His Gln Asp Asn Ala Glu Ser Ser Glu Lys
1               5                   10                  15


Arg Phe Arg Met Asn Ser Phe Val Ser Asp Phe Gly Arg Pro Leu Glu
            20                  25                  30


Pro Asp Lys Val Phe Ser Arg Gln Gly Asn Glu Glu Ser Arg Ser Leu
        35                  40                  45


Phe His Cys Tyr Ile Asn Glu Val Glu Arg Leu Asp Arg Ala Lys Ala
    50                  55                  60


Cys His Gln Thr Thr Ala Leu Asp Ser Asp Val Gln Leu Gln Glu Ala
65                  70                  75                  80
```

```
Ile Arg Ser Ser Gly Gln Pro Glu Glu Glu Leu Asn Arg Leu Met Lys
                85                  90                  95

Phe Asp Ile Pro Asn Phe Val Asn Thr Asp Gln Asn Tyr Phe Gly Glu
            100                 105                 110

Asp Asp Leu Leu Ile Ser Glu Pro Pro Ile Val Leu Glu Thr Lys Pro
        115                 120                 125

Leu Ser Gln Thr Ser His Lys Asp Leu Asp
    130                 135
```

```
<210>  13
<211>  126
<212>  DNA
<213>  Canis familiaris

<220>
<221>  CDS
<222>  (1)..(126)

<400>  13
atg tct gcc cag aga tta att tct aac aga aca tcc cag cca tct gca           48
Met Ser Ala Gln Arg Leu Ile Ser Asn Arg Thr Ser Gln Pro Ser Ala
1               5                   10                  15

cct aat tct gat tac acc tgg gaa tat gaa tat tat gaa att gga cca           96
Pro Asn Ser Asp Tyr Thr Trp Glu Tyr Glu Tyr Tyr Glu Ile Gly Pro
            20                  25                  30

gtg tcc ttt gaa gga ctg aag gct cat aaa                                  126
Val Ser Phe Glu Gly Leu Lys Ala His Lys
        35                  40
```

```
<210>  14
<211>  42
<212>  PRT
<213>  Canis familiaris

<400>  14

Met Ser Ala Gln Arg Leu Ile Ser Asn Arg Thr Ser Gln Pro Ser Ala
1               5                   10                  15

Pro Asn Ser Asp Tyr Thr Trp Glu Tyr Glu Tyr Tyr Glu Ile Gly Pro
            20                  25                  30

Val Ser Phe Glu Gly Leu Lys Ala His Lys
        35                  40
```

```
<210>  15
<211>  420
<212>  DNA
```

<213>  Canis familiaris

<220>
<221>  CDS
<222>  (1)..(420)

<400>  15

```
acc aag acg gga gct cca cac caa gac aat gca gaa tct tca gag aag       48
Thr Lys Thr Gly Ala Pro His Gln Asp Asn Ala Glu Ser Ser Glu Lys
1               5                   10                  15

aga ttt aga atg aat agc ttt gtg tca gac ttt gga aga cca ctg gag       96
Arg Phe Arg Met Asn Ser Phe Val Ser Asp Phe Gly Arg Pro Leu Glu
            20                  25                  30

cca gat aag gtg ttt tct cga cag ggc aat gat gaa tcc agg tct ctc      144
Pro Asp Lys Val Phe Ser Arg Gln Gly Asn Asp Glu Ser Arg Ser Leu
        35                  40                  45

ttt cat tgc tac atc aat gaa gtg gaa cac ttg gat agg gct aaa gtt      192
Phe His Cys Tyr Ile Asn Glu Val Glu His Leu Asp Arg Ala Lys Val
    50                  55                  60

tgt cat cag acc acg gtc ctt gac agc agt gtt cga ctc cag gaa gcc      240
Cys His Gln Thr Thr Val Leu Asp Ser Ser Val Arg Leu Gln Glu Ala
65                  70                  75                  80

att aga agc aat ggg cgt cca gag gag gag ctg aat agg ctt atg aag      288
Ile Arg Ser Asn Gly Arg Pro Glu Glu Glu Leu Asn Arg Leu Met Lys
                85                  90                  95

ttt gat atc cct aac ttt gtg aat aca gac cag aac tcc tcc ttt ggg      336
Phe Asp Ile Pro Asn Phe Val Asn Thr Asp Gln Asn Ser Ser Phe Gly
            100                 105                 110

gag gat gat ctt cta att tca gaa cca cct att gtt cta gaa aat aag      384
Glu Asp Asp Leu Leu Ile Ser Glu Pro Pro Ile Val Leu Glu Asn Lys
        115                 120                 125

cca gtt tcc cag acc tca cac aaa gac ctg gat tga                      420
Pro Val Ser Gln Thr Ser His Lys Asp Leu Asp
    130                 135
```

<210>  16
<211>  139
<212>  PRT
<213>  Canis familiaris

<400>  16

```
Thr Lys Thr Gly Ala Pro His Gln Asp Asn Ala Glu Ser Ser Glu Lys
1               5                   10                  15

Arg Phe Arg Met Asn Ser Phe Val Ser Asp Phe Gly Arg Pro Leu Glu
            20                  25                  30

Pro Asp Lys Val Phe Ser Arg Gln Gly Asn Asp Glu Ser Arg Ser Leu
        35                  40                  45
```

```
Phe His Cys Tyr Ile Asn Glu Val Glu His Leu Asp Arg Ala Lys Val
    50                  55                  60

Cys His Gln Thr Thr Val Leu Asp Ser Ser Val Arg Leu Gln Glu Ala
65                  70                  75                  80

Ile Arg Ser Asn Gly Arg Pro Glu Glu Glu Leu Asn Arg Leu Met Lys
                85                  90                  95

Phe Asp Ile Pro Asn Phe Val Asn Thr Asp Gln Asn Ser Ser Phe Gly
            100                 105                 110

Glu Asp Asp Leu Leu Ile Ser Glu Pro Pro Ile Val Leu Glu Asn Lys
        115                 120                 125

Pro Val Ser Gln Thr Ser His Lys Asp Leu Asp
    130                 135


<210>  17
<211>  126
<212>  DNA
<213>  Felis catus


<220>
<221>  CDS
<222>  (1)..(126)

<400>  17
atg tct gcc cag agg tta att tct aac aga aca tcc cag caa tct gca         48
Met Ser Ala Gln Arg Leu Ile Ser Asn Arg Thr Ser Gln Gln Ser Ala
1               5                   10                  15

tct aat tct gat tac acc tgg gaa tat gaa tat tat gag att gga cca         96
Ser Asn Ser Asp Tyr Thr Trp Glu Tyr Glu Tyr Tyr Glu Ile Gly Pro
            20                  25                  30

gtt tcc ttt gaa gga ctg aag gct cat aaa                                126
Val Ser Phe Glu Gly Leu Lys Ala His Lys
        35                  40


<210>  18
<211>  42
<212>  PRT
<213>  Felis catus

<400>  18

Met Ser Ala Gln Arg Leu Ile Ser Asn Arg Thr Ser Gln Gln Ser Ala
1               5                   10                  15

Ser Asn Ser Asp Tyr Thr Trp Glu Tyr Glu Tyr Tyr Glu Ile Gly Pro
            20                  25                  30
```

```
Val Ser Phe Glu Gly Leu Lys Ala His Lys
        35                  40


<210>  19
<211>  420
<212>  DNA
<213>  Felis catus


<220>
<221>  CDS
<222>  (1)..(420)

<400>  19
acc aag acg gga gct cca cac caa gac aat gca gag tct tca gag aag        48
Thr Lys Thr Gly Ala Pro His Gln Asp Asn Ala Glu Ser Ser Glu Lys
1               5                   10                  15

aga ttc aga atg aat agc ttt gtg tca gac ttc gga aga cca ctg gag        96
Arg Phe Arg Met Asn Ser Phe Val Ser Asp Phe Gly Arg Pro Leu Glu
            20                  25                  30

cca gat aag gtg ttt tct cga cag ggc aat gag gaa tcc agg tct ctc       144
Pro Asp Lys Val Phe Ser Arg Gln Gly Asn Glu Glu Ser Arg Ser Leu
        35                  40                  45

ttt cac tgc tac atc aac gaa gtg gaa cac ttg gat agg gct aaa gct       192
Phe His Cys Tyr Ile Asn Glu Val Glu His Leu Asp Arg Ala Lys Ala
    50                  55                  60

tgt cag cag acc aca gcc ctt gac agc tgt gtt caa ctg cag gaa gcc       240
Cys Gln Gln Thr Thr Ala Leu Asp Ser Cys Val Gln Leu Gln Glu Ala
65                  70                  75                  80

att aga agc aac ggg cgg cca gag gag gag ttg aac agg ctc atg aag       288
Ile Arg Ser Asn Gly Arg Pro Glu Glu Glu Leu Asn Arg Leu Met Lys
                85                  90                  95

ttt gac atc ccc aac ttc gtg aac aca gac cag aac tcc tcc ttt ggg       336
Phe Asp Ile Pro Asn Phe Val Asn Thr Asp Gln Asn Ser Ser Phe Gly
            100                 105                 110

gag gat gat ctt ctg att tca gaa cca cct att gtt cta gaa aat aag       384
Glu Asp Asp Leu Leu Ile Ser Glu Pro Pro Ile Val Leu Glu Asn Lys
        115                 120                 125

cca gtt tcc cag acg tca cac aaa gac ctg gat tga                       420
Pro Val Ser Gln Thr Ser His Lys Asp Leu Asp
    130                 135


<210>  20
<211>  139
<212>  PRT
<213>  Felis catus

<400>  20

Thr Lys Thr Gly Ala Pro His Gln Asp Asn Ala Glu Ser Ser Glu Lys
1               5                   10                  15
```

```
Arg Phe Arg Met Asn Ser Phe Val Ser Asp Phe Gly Arg Pro Leu Glu
            20                  25                  30

Pro Asp Lys Val Phe Ser Arg Gln Gly Asn Glu Glu Ser Arg Ser Leu
        35                  40                  45

Phe His Cys Tyr Ile Asn Glu Val Glu His Leu Asp Arg Ala Lys Ala
    50                  55                  60

Cys Gln Gln Thr Thr Ala Leu Asp Ser Cys Val Gln Leu Gln Glu Ala
65                  70                  75                  80

Ile Arg Ser Asn Gly Arg Pro Glu Glu Glu Leu Asn Arg Leu Met Lys
                85                  90                  95

Phe Asp Ile Pro Asn Phe Val Asn Thr Asp Gln Asn Ser Ser Phe Gly
            100                 105                 110

Glu Asp Asp Leu Leu Ile Ser Glu Pro Pro Ile Val Leu Glu Asn Lys
        115                 120                 125

Pro Val Ser Gln Thr Ser His Lys Asp Leu Asp
    130                 135


<210>  21
<211>  138
<212>  DNA
<213>  Mus musculus


<220>
<221>  CDS
<222>  (1)..(138)

<400>  21
atg gag atg tct gcc cag agg ctg gct tct aac agg aca tcc cca cag          48
Met Glu Met Ser Ala Gln Arg Leu Ala Ser Asn Arg Thr Ser Pro Gln
1               5                   10                  15

tca cca tcg aac tct gac tac acc tgg gaa tac gag tac tat gag atc          96
Ser Pro Ser Asn Ser Asp Tyr Thr Trp Glu Tyr Glu Tyr Tyr Glu Ile
            20                  25                  30

gga cca gtt tcc ttt gaa gga ctg aag gct cat aaa tat tcc                 138
Gly Pro Val Ser Phe Glu Gly Leu Lys Ala His Lys Tyr Ser
        35                  40                  45


<210>  22
<211>  46
<212>  PRT
<213>  Mus musculus

<400>  22
```

```
Met Glu Met Ser Ala Gln Arg Leu Ala Ser Asn Arg Thr Ser Pro Gln
1               5                   10                  15


Ser Pro Ser Asn Ser Asp Tyr Thr Trp Glu Tyr Glu Tyr Tyr Glu Ile
            20                  25                  30


Gly Pro Val Ser Phe Glu Gly Leu Lys Ala His Lys Tyr Ser
        35                  40                  45


<210>  23
<211>  423
<212>  DNA
<213>  Mus musculus


<220>
<221>  CDS
<222>  (1)..(423)

<400>  23
ttg ctg acg aag aca ggt gcc cca cac caa gac aac gcg gag tcc tca          48
Leu Leu Thr Lys Thr Gly Ala Pro His Gln Asp Asn Ala Glu Ser Ser
1               5                   10                  15

gag agg agg ttc cgc atg aac agc ttt gtg tca gac ttc ggg aag ccg          96
Glu Arg Arg Phe Arg Met Asn Ser Phe Val Ser Asp Phe Gly Lys Pro
            20                  25                  30

ctg gag tca gac aag gtg ttt tct cgt cag ggc aac gag gag tcc agg         144
Leu Glu Ser Asp Lys Val Phe Ser Arg Gln Gly Asn Glu Glu Ser Arg
        35                  40                  45

tct ctg ttc cac tgt tac atc aat gaa gta gaa cac ttg gac agg gtt         192
Ser Leu Phe His Cys Tyr Ile Asn Glu Val Glu His Leu Asp Arg Val
    50                  55                  60

aaa gtt tgc cac caa aca aca gcc atc gac agt gat gtc cac ctc cag         240
Lys Val Cys His Gln Thr Thr Ala Ile Asp Ser Asp Val His Leu Gln
65                  70                  75                  80

gaa gcc agc aga agc agt ggg agg cct gag gag gag cta gcc agg ttc         288
Glu Ala Ser Arg Ser Ser Gly Arg Pro Glu Glu Glu Leu Ala Arg Phe
                85                  90                  95

atg aag ttt gac atc ccc aac ttt gtg aac aca gag cag agc tcc ttt         336
Met Lys Phe Asp Ile Pro Asn Phe Val Asn Thr Glu Gln Ser Ser Phe
            100                 105                 110

ggg gag gat gat ctc ctg att tcg gaa gca ccc gtt ctt cta gaa aac         384
Gly Glu Asp Asp Leu Leu Ile Ser Glu Ala Pro Val Leu Leu Glu Asn
        115                 120                 125

aag cca gtt tcc cag aca tca cgc ata gac ctg gac tga                     423
Lys Pro Val Ser Gln Thr Ser Arg Ile Asp Leu Asp
    130                 135                 140


<210>  24
<211>  140
```

<212> PRT
<213> Mus musculus

<400> 24

Leu Leu Thr Lys Thr Gly Ala Pro His Gln Asp Asn Ala Glu Ser Ser
1               5                   10                  15

Glu Arg Arg Phe Arg Met Asn Ser Phe Val Ser Asp Phe Gly Lys Pro
            20                  25                  30

Leu Glu Ser Asp Lys Val Phe Ser Arg Gln Gly Asn Glu Glu Ser Arg
        35                  40                  45

Ser Leu Phe His Cys Tyr Ile Asn Glu Val Glu His Leu Asp Arg Val
    50                  55                  60

Lys Val Cys His Gln Thr Thr Ala Ile Asp Ser Asp Val His Leu Gln
65                  70                  75                  80

Glu Ala Ser Arg Ser Ser Gly Arg Pro Glu Glu Glu Leu Ala Arg Phe
                85                  90                  95

Met Lys Phe Asp Ile Pro Asn Phe Val Asn Thr Glu Gln Ser Ser Phe
            100                 105                 110

Gly Glu Asp Asp Leu Leu Ile Ser Glu Ala Pro Val Leu Leu Glu Asn
        115                 120                 125

Lys Pro Val Ser Gln Thr Ser Arg Ile Asp Leu Asp
    130                 135                 140

<210> 25
<211> 20
<212> DNA
<213> Artificial

<220>
<223> T3 primer

<400> 25
aattaaccct cactaaaggg 20

<210> 26
<211> 19
<212> DNA
<213> Artificial

<220>
<223> T7 primer

<400> 26
taatacgact cactatagg 19

<210> 27
<211> 22
<212> DNA
<213> Artificial

<220>
<223> canis RT primer sense

<400> 27
tcgacagggc aatgatgaat cc 22

<210> 28
<211> 21
<212> DNA
<213> Artificial

<220>
<223> canis RT primer antisense

<400> 28
tggacgccca ttgcttctaa t 21

<210> 29
<211> 22
<212> DNA
<213> Artificial

<220>
<223> human RT primer sense

<400> 29
ttgggttggt cttgcagtct tc 22

<210> 30
<211> 21
<212> DNA
<213> Artificial

<220>
<223> human RT primer antisense

<400> 30
tccaagcgtt ccacctcatt g 21

<210> 31
<211> 20
<212> DNA
<213> Artificial

<220>
<223> mouse RT primer sense

<400> 31
ctccaggaag ccagcagaag 20

<210> 32

<211>  21
<212>  DNA
<213>  Artificial

<220>
<223>  mouse RT primer antisense

<400>  32
caaaggagct ctgctctgtg t                                                21

<210>  33
<211>  35
<212>  DNA
<213>  Artificial

<220>
<223>  human MRAP2 EcoRI primer sense

<400>  33
gaattcgccg ccaccatgtc cgcccagagg ttaat                                 35

<210>  34
<211>  31
<212>  DNA
<213>  Artificial

<220>
<223>  human MRAP2 NotI primer antisense

<400>  34
gcggccgctc aatccaggtc tttgtgtgag g                                     31

<210>  35
<211>  26
<212>  DNA
<213>  Artificial

<220>
<223>  human N.MRAP2ECD KpnI primer sense

<400>  35
ggtaccatgt ccgcccagag gttaat                                           26

<210>  36
<211>  42
<212>  DNA
<213>  Artificial

<220>
<223>  human N.MRAP2ECD EcoRI primer antisense

<400>  36
gaattcgggg aatatttatg agccttcagt ccttcaaagg aa                         42

<210>  37
<211>  849
<212>  DNA
<213>  Artificial

```
<220>
<223>  Fc fusion protein


<220>
<221>  CDS
<222>  (1)..(849)

<400>  37
atg tcc gcc cag agg tta att tct aac aga acc tcc cag caa tcg gca        48
Met Ser Ala Gln Arg Leu Ile Ser Asn Arg Thr Ser Gln Gln Ser Ala
1               5                   10                  15

tct aat tct gat tac acc tgg gaa tat gaa tat tat gag att gga cca        96
Ser Asn Ser Asp Tyr Thr Trp Glu Tyr Glu Tyr Tyr Glu Ile Gly Pro
            20                  25                  30

gtt tcc ttt gaa gga ctg aag gct cat aaa tat tcc ccg aat tct gca       144
Val Ser Phe Glu Gly Leu Lys Ala His Lys Tyr Ser Pro Asn Ser Ala
        35                  40                  45

gat atc ccc aga ggg ccc aca atc aag ccc tgt cct cca tgc aaa tgc       192
Asp Ile Pro Arg Gly Pro Thr Ile Lys Pro Cys Pro Pro Cys Lys Cys
    50                  55                  60

cca gca cct aac ctc ttg ggt gga cca tcc gtc ttc atc ttc cct cca       240
Pro Ala Pro Asn Leu Leu Gly Gly Pro Ser Val Phe Ile Phe Pro Pro
65                  70                  75                  80

aag atc aag gat gta ctc atg atc tcc ctg agc ccc ata gtc aca tgt       288
Lys Ile Lys Asp Val Leu Met Ile Ser Leu Ser Pro Ile Val Thr Cys
                85                  90                  95

gtg gtg gtg gat gtg agc gag gat gac cca gat gtc cag atc agc tgg       336
Val Val Val Asp Val Ser Glu Asp Asp Pro Asp Val Gln Ile Ser Trp
            100                 105                 110

ttt gtg aac aac gtg gaa gta cac aca gct cag aca caa acc cat aga       384
Phe Val Asn Asn Val Glu Val His Thr Ala Gln Thr Gln Thr His Arg
        115                 120                 125

gag gat tac aac agt act ctc cgg gtg gtc agt gcc ctc ccc atc cag       432
Glu Asp Tyr Asn Ser Thr Leu Arg Val Val Ser Ala Leu Pro Ile Gln
    130                 135                 140

cac cag gac tgg atg agt ggc aag gag ttc aaa tgc aag gtc aac aac       480
His Gln Asp Trp Met Ser Gly Lys Glu Phe Lys Cys Lys Val Asn Asn
145                 150                 155                 160

aaa gac ctc cca gcg ccc atc gag aga acc atc tca aaa ccc aaa ggg       528
Lys Asp Leu Pro Ala Pro Ile Glu Arg Thr Ile Ser Lys Pro Lys Gly
                165                 170                 175

tca gta aga gct cca cag gta tat gtc ttg cct cca cca gaa gaa gag       576
Ser Val Arg Ala Pro Gln Val Tyr Val Leu Pro Pro Pro Glu Glu Glu
            180                 185                 190

atg act aag aaa cag gtc act ctg acc tgc atg gtc aca gac ttc atg       624
Met Thr Lys Lys Gln Val Thr Leu Thr Cys Met Val Thr Asp Phe Met
        195                 200                 205

cct gaa gac att tac gtg gag tgg acc aac aac ggg aaa aca gag cta       672
```

```
Pro Glu Asp Ile Tyr Val Glu Trp Thr Asn Asn Gly Lys Thr Glu Leu
    210                 215                 220

aac tac aag aac act gaa cca gtc ctg gac tct gat ggt tct tac ttc      720
Asn Tyr Lys Asn Thr Glu Pro Val Leu Asp Ser Asp Gly Ser Tyr Phe
225                 230                 235                 240

atg tac agc aag ctg aga gtg gaa aag aag aac tgg gtg gaa aga aat      768
Met Tyr Ser Lys Leu Arg Val Glu Lys Lys Asn Trp Val Glu Arg Asn
                245                 250                 255

agc tac tcc tgt tca gtg gtc cac gag ggt ctg cac aat cac cac acg      816
Ser Tyr Ser Cys Ser Val Val His Glu Gly Leu His Asn His His Thr
            260                 265                 270

act aag agc ttc tcc cgg act ccg ggt aaa tga                          849
Thr Lys Ser Phe Ser Arg Thr Pro Gly Lys
        275                 280


<210>  38
<211>  282
<212>  PRT
<213>  Artificial

<220>
<223>  Fc fusion protein

<400>  38

Met Ser Ala Gln Arg Leu Ile Ser Asn Arg Thr Ser Gln Gln Ser Ala
1               5                   10                  15


Ser Asn Ser Asp Tyr Thr Trp Glu Tyr Glu Tyr Tyr Glu Ile Gly Pro
            20                  25                  30


Val Ser Phe Glu Gly Leu Lys Ala His Lys Tyr Ser Pro Asn Ser Ala
        35                  40                  45


Asp Ile Pro Arg Gly Pro Thr Ile Lys Pro Cys Pro Pro Cys Lys Cys
    50                  55                  60


Pro Ala Pro Asn Leu Leu Gly Gly Pro Ser Val Phe Ile Phe Pro Pro
65                  70                  75                  80


Lys Ile Lys Asp Val Leu Met Ile Ser Leu Ser Pro Ile Val Thr Cys
                85                  90                  95


Val Val Val Asp Val Ser Glu Asp Asp Pro Asp Val Gln Ile Ser Trp
            100                 105                 110


Phe Val Asn Asn Val Glu Val His Thr Ala Gln Thr Gln Thr His Arg
        115                 120                 125


Glu Asp Tyr Asn Ser Thr Leu Arg Val Val Ser Ala Leu Pro Ile Gln
```

130 135 140

His Gln Asp Trp Met Ser Gly Lys Glu Phe Lys Cys Lys Val Asn Asn
145 150 155 160

Lys Asp Leu Pro Ala Pro Ile Glu Arg Thr Ile Ser Lys Pro Lys Gly
165 170 175

Ser Val Arg Ala Pro Gln Val Tyr Val Leu Pro Pro Pro Glu Glu Glu
180 185 190

Met Thr Lys Lys Gln Val Thr Leu Thr Cys Met Val Thr Asp Phe Met
195 200 205

Pro Glu Asp Ile Tyr Val Glu Trp Thr Asn Asn Gly Lys Thr Glu Leu
210 215 220

Asn Tyr Lys Asn Thr Glu Pro Val Leu Asp Ser Asp Gly Ser Tyr Phe
225 230 235 240

Met Tyr Ser Lys Leu Arg Val Glu Lys Lys Asn Trp Val Glu Arg Asn
245 250 255

Ser Tyr Ser Cys Ser Val Val His Glu Gly Leu His Asn His His Thr
260 265 270

Thr Lys Ser Phe Ser Arg Thr Pro Gly Lys
275 280

<210> 39
<211> 24
<212> DNA
<213> Artificial

<220>
<223> human C.MRAP2ECD KpnI primer sense

<400> 39
ggtaccacca agacaggagc ccca 24

<210> 40
<211> 30
<212> DNA
<213> Artificial

<220>
<223> human C.MRAP2ECD EcoRI primer antisense

<400> 40
gaattcggat ccaggtcttt gtgtgaggtc 30

<210> 41
<211> 1131
<212> DNA
<213> Artificial

<220>
<223> Fc fusion protein

<220>
<221> CDS
<222> (1)..(1131)

<400> 41

```
acc aag aca gga gcc cca cac caa gac aat gca gag tcc tca gag aag      48
Thr Lys Thr Gly Ala Pro His Gln Asp Asn Ala Glu Ser Ser Glu Lys
1               5                   10                  15

aga ttc aga atg aac agc ttt gtg tca gac ttt gga aga cct ctg gag      96
Arg Phe Arg Met Asn Ser Phe Val Ser Asp Phe Gly Arg Pro Leu Glu
            20                  25                  30

cca gat aaa gta ttt tct cgc caa ggc aac gag gag tcc agg tct ctc      144
Pro Asp Lys Val Phe Ser Arg Gln Gly Asn Glu Glu Ser Arg Ser Leu
        35                  40                  45

ttt cac tgc tac atc aat gag gtg gaa cgc ttg gac aga gcc aaa gct      192
Phe His Cys Tyr Ile Asn Glu Val Glu Arg Leu Asp Arg Ala Lys Ala
    50                  55                  60

tgt cac cag acc aca gcc ctt gac agt gac gtc caa ctc cag gaa gcc      240
Cys His Gln Thr Thr Ala Leu Asp Ser Asp Val Gln Leu Gln Glu Ala
65                  70                  75                  80

atc aga agc agt ggg cag cca gag gag gag ctg aac agg ctc atg aag      288
Ile Arg Ser Ser Gly Gln Pro Glu Glu Glu Leu Asn Arg Leu Met Lys
                85                  90                  95

ttt gac atc ccc aac ttt gtg aac aca gac cag aac tac ttt ggg gag      336
Phe Asp Ile Pro Asn Phe Val Asn Thr Asp Gln Asn Tyr Phe Gly Glu
            100                 105                 110

gat gat ctt ctg att tct gaa cca cct att gtt ctg gaa act aag cca      384
Asp Asp Leu Leu Ile Ser Glu Pro Pro Ile Val Leu Glu Thr Lys Pro
        115                 120                 125

ctt tcc cag acc tca cac aaa gac ctg gat ccg aat tct gca gat atc      432
Leu Ser Gln Thr Ser His Lys Asp Leu Asp Pro Asn Ser Ala Asp Ile
    130                 135                 140

ccc aga ggg ccc aca atc aag ccc tgt cct cca tgc aaa tgc cca gca      480
Pro Arg Gly Pro Thr Ile Lys Pro Cys Pro Pro Cys Lys Cys Pro Ala
145                 150                 155                 160

cct aac ctc ttg ggt gga cca tcc gtc ttc atc ttc cct cca aag atc      528
Pro Asn Leu Leu Gly Gly Pro Ser Val Phe Ile Phe Pro Pro Lys Ile
                165                 170                 175

aag gat gta ctc atg atc tcc ctg agc ccc ata gtc aca tgt gtg gtg      576
Lys Asp Val Leu Met Ile Ser Leu Ser Pro Ile Val Thr Cys Val Val
            180                 185                 190

gtg gat gtg agc gag gat gac cca gat gtc cag atc agc tgg ttt gtg      624
```

```
Val Asp Val Ser Glu Asp Asp Pro Asp Val Gln Ile Ser Trp Phe Val
        195                 200                 205

aac aac gtg gaa gta cac aca gct cag aca caa acc cat aga gag gat        672
Asn Asn Val Glu Val His Thr Ala Gln Thr Gln Thr His Arg Glu Asp
    210                 215                 220

tac aac agt act ctc cgg gtg gtc agt gcc ctc ccc atc cag cac cag        720
Tyr Asn Ser Thr Leu Arg Val Val Ser Ala Leu Pro Ile Gln His Gln
225                 230                 235                 240

gac tgg atg agt ggc aag gag ttc aaa tgc aag gtc aac aac aaa gac        768
Asp Trp Met Ser Gly Lys Glu Phe Lys Cys Lys Val Asn Asn Lys Asp
                245                 250                 255

ctc cca gcg ccc atc gag aga acc atc tca aaa ccc aaa ggg tca gta        816
Leu Pro Ala Pro Ile Glu Arg Thr Ile Ser Lys Pro Lys Gly Ser Val
            260                 265                 270

aga gct cca cag gta tat gtc ttg cct cca cca gaa gaa gag atg act        864
Arg Ala Pro Gln Val Tyr Val Leu Pro Pro Pro Glu Glu Glu Met Thr
        275                 280                 285

aag aaa cag gtc act ctg acc tgc atg gtc aca gac ttc atg cct gaa        912
Lys Lys Gln Val Thr Leu Thr Cys Met Val Thr Asp Phe Met Pro Glu
    290                 295                 300

gac att tac gtg gag tgg acc aac aac ggg aaa aca gag cta aac tac        960
Asp Ile Tyr Val Glu Trp Thr Asn Asn Gly Lys Thr Glu Leu Asn Tyr
305                 310                 315                 320

aag aac act gaa cca gtc ctg gac tct gat ggt tct tac ttc atg tac       1008
Lys Asn Thr Glu Pro Val Leu Asp Ser Asp Gly Ser Tyr Phe Met Tyr
                325                 330                 335

agc aag ctg aga gtg gaa aag aag aac tgg gtg gaa aga aat agc tac       1056
Ser Lys Leu Arg Val Glu Lys Lys Asn Trp Val Glu Arg Asn Ser Tyr
            340                 345                 350

tcc tgt tca gtg gtc cac gag ggt ctg cac aat cac cac acg act aag       1104
Ser Cys Ser Val Val His Glu Gly Leu His Asn His His Thr Thr Lys
        355                 360                 365

agc ttc tcc cgg act ccg ggt aaa tga                                   1131
Ser Phe Ser Arg Thr Pro Gly Lys
    370                 375


<210>  42
<211>  376
<212>  PRT
<213>  Artificial

<220>
<223>  Fc fusion protein

<400>  42

Thr Lys Thr Gly Ala Pro His Gln Asp Asn Ala Glu Ser Ser Glu Lys
1               5                   10                  15

Arg Phe Arg Met Asn Ser Phe Val Ser Asp Phe Gly Arg Pro Leu Glu
```

20 25 30

Pro Asp Lys Val Phe Ser Arg Gln Gly Asn Glu Glu Ser Arg Ser Leu
35 40 45

Phe His Cys Tyr Ile Asn Glu Val Glu Arg Leu Asp Arg Ala Lys Ala
50 55 60

Cys His Gln Thr Thr Ala Leu Asp Ser Asp Val Gln Leu Gln Glu Ala
65 70 75 80

Ile Arg Ser Ser Gly Gln Pro Glu Glu Glu Leu Asn Arg Leu Met Lys
85 90 95

Phe Asp Ile Pro Asn Phe Val Asn Thr Asp Gln Asn Tyr Phe Gly Glu
100 105 110

Asp Asp Leu Leu Ile Ser Glu Pro Pro Ile Val Leu Glu Thr Lys Pro
115 120 125

Leu Ser Gln Thr Ser His Lys Asp Leu Asp Pro Asn Ser Ala Asp Ile
130 135 140

Pro Arg Gly Pro Thr Ile Lys Pro Cys Pro Pro Cys Lys Cys Pro Ala
145 150 155 160

Pro Asn Leu Leu Gly Gly Pro Ser Val Phe Ile Phe Pro Pro Lys Ile
165 170 175

Lys Asp Val Leu Met Ile Ser Leu Ser Pro Ile Val Thr Cys Val Val
180 185 190

Val Asp Val Ser Glu Asp Asp Pro Asp Val Gln Ile Ser Trp Phe Val
195 200 205

Asn Asn Val Glu Val His Thr Ala Gln Thr Gln Thr His Arg Glu Asp
210 215 220

Tyr Asn Ser Thr Leu Arg Val Val Ser Ala Leu Pro Ile Gln His Gln
225 230 235 240

Asp Trp Met Ser Gly Lys Glu Phe Lys Cys Lys Val Asn Asn Lys Asp
245 250 255

Leu Pro Ala Pro Ile Glu Arg Thr Ile Ser Lys Pro Lys Gly Ser Val
260 265 270

```
Arg Ala Pro Gln Val Tyr Val Leu Pro Pro Pro Glu Glu Glu Met Thr
        275                 280                 285

Lys Lys Gln Val Thr Leu Thr Cys Met Val Thr Asp Phe Met Pro Glu
    290                 295                 300

Asp Ile Tyr Val Glu Trp Thr Asn Asn Gly Lys Thr Glu Leu Asn Tyr
305                 310                 315                 320

Lys Asn Thr Glu Pro Val Leu Asp Ser Asp Gly Ser Tyr Phe Met Tyr
                325                 330                 335

Ser Lys Leu Arg Val Glu Lys Lys Asn Trp Val Glu Arg Asn Ser Tyr
            340                 345                 350

Ser Cys Ser Val Val His Glu Gly Leu His Asn His His Thr Thr Lys
        355                 360                 365

Ser Phe Ser Arg Thr Pro Gly Lys
    370                 375
```

## Description

Technical Field

The present invention relates to a novel medical use of antibodies to MRAP2 or fragments thereof as, for example, therapeutic and/or preventive agents for cancer.

Background Art

In recent years, a variety of antibody medicines for cancer treatment that target antigen proteins on cancer cells have come into existence. The antibody medicines used as cancer-specific therapeutic agents exhibit drug efficacy to a certain extent, and thus they have been gaining attention. However, many of target antigen proteins are also expressed on multiple normal cells. As a result of antibody administration, not only cancer cells, but also normal cells on which a target antigen has been expressed can be damaged, thereby causing a side effect, which becomes problematic. Hence, it is expected that, if it becomes possible to identify cancer antigens that are specifically expressed on the surface of a cancer cell and to use antibodies targeting such antigens as medicaments, then treatment with antibody medicines that cause fewer side effects could be realized.

Melanocortin 2 receptor accessory protein 2 (MRAP2), a type 1 or type 2 transmembrane protein, participates in the control of melanocortin receptor (MCR) activity and functions in energy metabolism *in vivo* (Non Patent Literature 1). Also, it has been reported that MRAP2-deficient mice become obese in spite of the absence of overeating, and it has also been reported as to humans that some severely obese patients have a mutation in the MRAP2 gene (Non Patent Literature 2). However, none of the previous reports show that the MRAP2 protein has immunity-inducing activity against cancer cells and is thereby useful for treatment or prevention of cancers.

Prior Art Literatures

Non Patent Literatures

Non Patent Literature 1: Jackson DS. et al., Front. Neurosci, 9:213 (2015)
Non Patent Literature 2: Asai M. et al., Science, 341:275-278 (2013)


Summary of Invention

Technical Problem

An object of the present invention is to identify cancer antigen proteins specifically expressed on the surface of cancer cells and to provide a use of antibodies targeting such proteins as therapeutic and/or preventive agents for cancer.

Solution to Problem

As a result of intensive studies, the present inventors have now obtained cDNA encoding a protein that binds to an antibody present in the serum from a tumor-bearing organism by the SEREX method using canine testis tissue-derived cDNA libraries and sera from dogs with leukemia. With the use of the obtained canine genes and gene homologs from human, feline, and mouse, MRAP2 proteins having amino acid sequences shown in SEQ ID NO: 2, 4, 6 or 8 and antibodies against the MRAP2 proteins have now been prepared. In addition, the present inventors have now found that MRAP2 is specifically expressed in the cells of leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, or perianal adenocarcinoma, and that portions of the MRAP2 proteins are specifically expressed on the surface of such cancer cells. Further, the present inventors have now found that antibodies against the MRAP2 portions expressed on cancer cell surfaces can damage cancer cells expressing MRAP2. These findings have led to the completion of the present invention.

Therefore, the present invention includes (1) to (11) below.

(1) A pharmaceutical composition for treatment and/or prevention of a cancer, which comprises, as an active ingredient, an antibody or fragment thereof having an immunological reactivity with an MRAP2 protein having the amino acid sequence shown in SEQ ID NO: 2, 4, 6, or 8 or an amino acid sequence having 80% or more sequence identity with the amino acid sequence, or with a fragment of the MRAP2 protein comprising 7 or more consecutive amino acids.

(2) The pharmaceutical composition according to (1), which comprises, as an active ingredient, an antibody or fragment thereof having an immunological reactivity with a partial polypeptide of the MRAP2 protein, the partial polypeptide being a polypeptide consisting of 7 or more consecutive amino acids of the amino acid sequence shown in any one of the even numbered SEQ ID NOS: 10 to 24, or a polypeptide consisting of an amino acid sequence having 80% or more sequence identity with the amino acid sequence.

(3) The pharmaceutical composition according to (1) or (2), wherein the cancer is a cancer expressing MRAP2 on a cell surface.

(4) The pharmaceutical composition according to any one of (1) to (3), wherein the cancer is selected from the group consisting of leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, and perianal adenocarcinoma.

(5) The pharmaceutical composition according to any one of (1) to (4), wherein the antibody is a monoclonal or polyclonal antibody.

(6) The pharmaceutical composition according to any one of (1) to (5), wherein the antibody is a human antibody, a humanized antibody, a chimeric antibody, a single chain antibody, or a multispecific antibody.

(7) An antibody or fragment thereof having an immunological reactivity with an N-terminal partial polypeptide of an MRAP2 protein, the partial polypeptide being a polypeptide consisting of the amino acid sequence shown in SEQ ID NO: 10, 14, 18, or 22 or an amino acid sequence having 80% or more sequence identity with the amino acid sequence.

(8) An antibody or fragment thereof having an immunological reactivity with a C-terminal partial polypeptide of an MRAP2 protein, the partial polypeptide being a polypeptide consisting of the amino acid sequence shown in SEQ ID NO: 12, 16, 20, or 24 or an amino acid sequence having 80% or more sequence identity with the amino acid sequence.

(9) The antibody or fragment thereof according to (7) or (8), wherein the antibody is a human antibody, a humanized antibody, a chimeric antibody, a single chain antibody, or a multispecific antibody.

(10) A pharmaceutical combination for treatment and/or prevention of a cancer, which comprises the pharmaceutical composition according to any one of (1) to (6) and a pharmaceutical composition comprising an antitumor agent.

(11) A method for treating and/or preventing a cancer, which comprises administering, to a subject, an antibody or fragment thereof having an immunological reactivity with an MRAP2 protein having the amino acid sequence shown in SEQ ID NO: 2, 4, 6, or 8 or an amino acid sequence having 80% or more sequence identity with the amino acid sequence, or with a fragment of the MRAP2 protein comprising 7 or more consecutive amino acids.

This description includes all or part of the contents disclosed in Japanese Patent Application No. 2016-064033, to which the present application claims the priority.

Advantageous Effects of Invention

Antibodies against MRAP2 used in the present invention damage cancer cells. Therefore, such antibodies against MRAP2 are useful for treatment or prevention of cancers.

Brief Description of Drawings

Fig. 1 shows expression patterns of the identified canine MRAP2 gene in canine tumor tissues.

Fig. 2 shows expression patterns of the identified MRAP2 gene in each of human tissues and cancer cell lines. Fig. 2A shows the expression patterns of the human MRAP2 gene in each of human tissues. Fig. 2B shows the expression patterns of the human MRAP2 gene in each of human cancer cell lines.

Fig. 3 shows expression patterns of the identified mouse MRAP2 gene in each of mouse cancer cell lines.

Fig. 4 shows the cytotoxic activity of polyclonal antibodies to MRAP2 (anti-N-terminal portion of MRAP2 polyclonal antibody and anti-C-terminal portion of MRAP2 polyclonal antibody) against the leukemia cell line (K562) and the malignant lymphoma cell line (Namalwa) expressing MRAP2 gene. In this figure, Control-1 shows the cytotoxic activity against the K562 cells after addition of a control polyclonal antibody, Anti-N-terminal portion-1 shows the cytotoxic activity against the K562 cells after addition of the anti-N-terminal portion of MRAP2 polyclonal antibody, and Anti-C-terminal portion-1 shows the cytotoxic activity against the K562 cells after addition of the anti-C-terminal portion of MRAP2 polyclonal antibody. Control-2 shows the cytotoxic activity against the Namalwa cells after addition of the control polyclonal antibody, Anti-N-terminal portion-2 shows the cytotoxic activity against the Namalwa cells after addition of the anti-N-terminal portion of MRAP2 polyclonal antibody, and Anti-C-terminal portion-2 shows the cytotoxic activity against the Namalwa cells after addition of the anti-C-terminal portion of MRAP2 polyclonal antibody.

Description of Embodiments

The present invention relates to a use of an antibody or fragment (preferably antigen binding fragment) thereof to an MRAP2 protein or a fragment thereof for treatment and/or prevention of cancers.

The present invention relates to a pharmaceutical composition for treatment and/or prevention of a cancer, which comprises, as an active ingredient, an antibody or fragment thereof having an immunological reactivity with an MRAP2 protein having the amino acid sequence shown in SEQ ID NO: 2, 4, 6, or 8 or an amino acid sequence having 80% or more (preferably 85% or more, more preferably 90% or more, further preferably 95% or more, and particularly preferably 99% or more, for example, 99.5% or more) sequence identity with the amino acid sequence, or with a fragment of the MRAP2 protein comprising 7 or more (7 to each full-length sequence, preferably 7 to 150 and more preferably 7 to 50) consecutive amino acids.

The present invention also relates to the pharmaceutical composition for treatment and/or prevention of a cancer, which comprises, as an active ingredient, an antibody or fragment thereof having an immunological reactivity with a partial polypeptide of an MRAP2 protein, the partial polypeptide being a polypeptide consisting of 7 or more (7 to each full-length sequence, preferably 7 to 40, more preferably 7 to 20, for example, 7 to 12 or 8 to 11) consecutive amino acids of the amino acid sequence shown in any one of the even numbered SEQ ID NOS: 10 to 24, or a polypeptide consisting of an amino acid sequence having 80% or more (preferably 85% or more, more preferably 90% or more, further preferably 95% or more, and particularly preferably 97% or more) sequence identity with the amino acid sequence.

The antitumor activity of the antibody or fragment thereof to the polypeptide consisting of the amino acid sequence shown in SEQ ID NO: 2, 4, 6, or 8 or to a fragment of the polypeptide used in the present invention can be evaluated by examining *in vivo* the inhibition of tumor growth in a tumor-bearing animal, or, as described below, by examining *in vitro* whether or not immunocyte- or complement-mediated cytotoxic activity against tumor cells expressing the polypeptide is exhibited. Likewise, the antitumor activity of the antibody or fragment thereof against the polypeptide consisting of the amino acid sequence shown in any one of the even numbered SEQ ID NOS: 10 to 24 or a fragment of the polypeptide used in the present invention can be evaluated by examining *in vivo* the inhibition of tumor growth in a tumor-bearing animal, or, as described below, by examining *in vitro* whether or not immunocyte- or complement-mediated cytotoxic activity against tumor cells expressing the polypeptide is exhibited.

In addition, the nucleotide sequences of polynucleotides encoding the proteins consisting of the amino acid sequences shown in SEQ ID NOS: 2, 4, 6, 8, 10, 12, 14, 16, 18, 20, 22, and 24 are shown in SEQ ID NOS: 1, 3, 5, 7, 9, 11, 13, 15, 17, 19, 21, and 23, respectively.

The amino acid sequence shown in SEQ ID NO: 4 in the Sequence Listing disclosed according to the present invention is the amino acid sequence of the MRAP2, which was isolated, by the SEREX method using canine testis tissue-derived cDNA libraries and sera from dogs with leukemia, as a polypeptide capable of binding to antibodies specifically existing in the sera from tumor-bearing dogs; the amino acid sequence shown in SEQ ID NO: 2 is the amino acid sequence of the MRAP2 isolated as a human homolog of said dog polypeptide; the amino acid sequence shown in SEQ ID NO: 6 is the amino acid sequence of the MRAP2 isolated as a feline homolog of said dog polypeptide; and the amino acid sequence shown in SEQ ID NO: 8 is the amino acid sequence of the MRAP2 protein isolated as a mouse homolog of said dog polypeptide (see Example 1 described below).

According to the present invention, an antibody that binds to a portion expressed on cancer cell surfaces within MRAP2 protein is preferably used. Specific examples thereof include antibodies to polypeptides having the amino acid sequence shown in SEQ ID NO: 10 (human), 14 (canine), 18 (feline), or 22 (mouse), which is the N-terminal portion of the MRAP2 protein, or the amino acid sequence shown in SEQ ID NO: 12 (human), 16 (canine), 20 (feline), or 24 (mouse), which is the C-terminal portion of the MRAP2 protein, or fragments of the polypeptides (preferably, the fragments each consisting of 7 or more consecutive amino acids of any one of the amino acid sequences), or polypeptides having an amino acid sequence having 80% or more, preferably 85% or more, more preferably 90% or more, further preferably 95% or more, and particularly preferably 99% or more sequence identity to any one of these polypeptides. Antibodies of the present invention include all antibodies capable of binding to the above polypeptides and having antitumor activity.

The antibodies to MRAP2 usable in the present invention as described above may be any types thereof, as long as they can exhibit antitumor activity. Examples thereof can include monoclonal antibodies, polyclonal antibodies, synthetic antibodies, multispecific antibodies (e.g., bispecific antibodies), human antibodies, humanized antibodies, chimeric antibodies, and single-chain antibodies (scFV). The antibodies used in the present invention also include antibody fragments, for example, antigen binding fragments such as Fab and $F(ab')_2$. These antibodies and fragments thereof can be prepared by methods known to persons skilled in the art. In the present invention, antibodies capable of specifically binding to an MRAP2 protein or fragments thereof are desirable. Such antibodies are preferably monoclonal antibodies; however, as long as homogenous antibodies can be stably produced, polyclonal antibodies may also be used. In addition, if the subject is a human, a human antibody or a humanized antibody is desirable in order to avoid or inhibit the immunorejection.

The word "specifically binding to an MRAP2 protein or fragments thereof" as used herein means that an antibody of interest specifically binds to the MRAP2 protein or fragments thereof and does not substantially bind to other proteins.

The antitumor activity of an antibody used in the present invention can be evaluated by examining *in vivo* the inhibition of tumor growth in a tumor-bearing animal, or, as described below, examining *in vitro* whether or not the immunocyte- or complement-mediated cytotoxic activity against tumor cells expressing the polypeptide is exhibited.

Moreover, the subjects in need of treatment and/or prevention of cancer according to the present invention are mammals such as human, pet animals, livestock animals, sport animals, or experimental animals. The preferred subject is a human.

Production of antigens, production of antibodies, and pharmaceutical compositions, related to the present invention, will be explained below.

<Production of antigens used for antibody production>

Proteins or fragments thereof used as sensitizing antigens for obtaining antibodies to MRAP2 used in the present invention are not limited in terms of their origins such as animals including, for example, humans, canines, felines, mice, bovines, horses, rats, and chickens. However, such proteins or fragments thereof are preferably selected in view of compatibility with parent cells used for cell fusion. Mammal-derived proteins are generally preferable and human-derived proteins are particularly preferable. For instance, if the MRAP2 is human MRAP2, a human MRAP2 protein, a partial polypeptide thereof, or cells capable of expressing human MRAP2 can be used.

Nucleotide sequences and amino acid sequences of human MRAP2 and homologs thereof can be obtained by, for example, accessing the website of GenBank (NCBI, USA) and using an algorithm such as BLAST or FASTA (Karlin and Altschul, Proc. Natl. Acad. Sci. USA, 90:5873-5877,1993; Altschul et al., Nucleic Acids Res. 25:3389-3402, 1997).

According to the present invention, when the nucleotide sequence (SEQ ID NO: 1) or the amino acid sequence (SEQ ID NO: 2) of human MRAP2 is used as a base sequence, targets are nucleic acids or proteins each consisting of a sequence having 70% to 100%, preferably 80% to 100%, more preferably 90% to 100%, and further preferably 95% to 100% (e.g., 97% to 100%, 98% to 100%, 99% to 100%, or 99.5% to 100%) sequence identity with the nucleotide sequence or amino acid sequence of the ORF or mature portion of the base nucleotide sequence or amino acid sequence. The term "% sequence identity" as used herein means a percentage (%) of the number of identical amino acids (or nucleotides) relative to the total number of amino acids (or nucleotides) in the case that two sequences are aligned such that maximum similarity can be achieved with or without introduction of gaps.

Fragments of an MRAP2 protein have lengths ranging from the amino acid length of an epitope (or an antigenic determinant), which is the smallest unit of an antigen recognized by an antibody, to less than the full-length of the protein. The epitope refers to a polypeptide fragment having antigenicity or immunogenicity in mammals and preferably in humans. The smallest unit of the epitope consists of approximately 7 to 12 amino acids, and for example, 8 to 11 amino acids. A specific example thereof is a polypeptide consisting of the amino acid sequence having 80% or more, preferably 85% or more, more preferably 90% or more, and further preferably 95% or more sequence identity with the amino acid sequence of an MRAP2 protein.

Polypeptides comprising the aforementioned human MRAP2 protein and partial peptides thereof can be synthesized according to chemical synthesis methods such as the Fmoc method (fluorenylmethyloxycarbonyl method) or the tBoc method (t-butyloxycarbonyl method) (the Japanese Biochemical Society (ed.), "Biochemical Experimentation Course (Seikagaku Jikken Koza) 1," Protein Chemistry IV, Chemical Modification and Peptide Synthesis, Kagaku-dojin Publishing Company, Inc. (Japan), 1981). Also, they can be synthesized by general methods using a variety of commercially available peptide synthesizers. In addition, polypeptides of interest can be obtained by preparing polynucleotides encoding the above polypeptides, incorporating each of the polynucleotides into an expression vector and introducing the vector into a host cell, thereby allowing the host cell to produce the polypeptide, using known gene engineering methods (Sambrook et al., Molecular Cloning, 2nd edition, Current Protocols in Molecular Biology (1989), Cold Spring Harbor Laboratory Press; Ausubel et al., Short Protocols in Molecular Biology, 3rd edition, A Compendium of Methods from Current Protocols in Molecular Biology (1995), John Wiley & Sons, etc.).

Polynucleotides encoding the aforementioned polypeptides can be readily prepared by known gene engineering techniques or general methods using commercially available nucleic acid synthesizers. For example, DNA comprising the nucleotide sequence shown in SEQ ID NO: 1 can be prepared by PCR using a human chromosome DNA or cDNA library as a template and a pair of primers designed to enable the amplification of the nucleotide sequence shown in SEQ ID NO: 1. PCR conditions can be appropriately determined. For example, such conditions may comprise conducting 30 cycles of the reaction steps consisting of: 94°C, 30 seconds (denaturation); 55°C, 30 seconds to 1 minute (annealing);

and 72°C, 1 minute (elongation) using a thermostable DNA polymerase (e.g., Taq polymerase) and a $Mg^{2+}$-containing PCR buffer, followed by reaction at 72°C for 7 minutes after completion of the 30 cycles. However, PCR conditions are not limited to the above-exemplified PCR conditions. PCR techniques and conditions are described in, for example, Ausubel et al., Short Protocols in Molecular Biology, 3rd edition, A Compendium of Methods from Current Protocols in Molecular Biology (1995), John Wiley & Sons (Chapter 15, in particular).

In addition, desired DNA can be isolated by preparing appropriate probes and primers based on information about the nucleotide and amino acid sequences shown in SEQ ID NOS: 1 to 8 in the Sequence Listing of the present application, and screening a cDNA library of e.g., human with the use of such probes and primers. Preferably, such cDNA library is produced from a cell, organ, or tissue in which the protein with SEQ ID NO: 2, 4, 6 or 8 is expressed. Examples of the cell or tissue include, but not limited to, cells or tissues from cancers or tumors, such as brain, leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, and perianal adenocarcinoma. Operations such as preparation of probes or primers, construction of cDNA libraries, screening of cDNA libraries, and cloning of genes of interest, as described above, are known to persons skilled in the art, and they can be carried out according to, for example, the methods described in Sambrook et al., Molecular Cloning, the 2nd edition, Current Protocols in Molecular Biology (1989) and Ausbel et al. (*ibid*.). DNAs encoding human MRAP2 protein and partial peptides thereof can be obtained from the thus obtained DNAs.

The above-described host cells may be any cells, as long as they can express the above-described polypeptides. An example of prokaryotic host cell includes, but is not limited to, *Escherichia coli.* Examples of eukaryotic host cells include, but are not limited to, mammalian cells such as monkey kidney cell (COS1), Chinese hamster ovary cell (CHO), human embryonic kidney cell line (HEK293), and mouse embryonic skin cell line (NIH3T3), yeast cells such as budding yeast and fission yeast cells, silkworm cells, and Xenopus *laevis* egg cells.

When prokaryotic cells are used as host cells, an expression vector preferably having an origin replicable in prokaryotic cells, a promoter, a ribosome-binding site, a multicloning site, a terminator, a drug resistance gene, an auxotrophic complementary gene, a reporter gene, or the like can be used. As expression vectors for *Escherichia coli,* pUC vectors, pBluescriptII, pET expression systems, pGEX expression systems, and the like can be exemplified. A DNA encoding the above polypeptide is incorporated into such an expression vector, a prokaryotic host cell is transformed with the vector, and then the thus obtained transformed cell is cultured, so that the polypeptide encoded by the DNA can be expressed in the prokaryotic host cell. At this time, the polypeptide can also be expressed as a fusion protein with another protein.

When eukaryotic cells are used as host cells, expression vectors for eukaryotic cells preferably having a promoter, a splicing region, a poly(A) addition site, or the like can be used. Examples of such expression vectors include pKA1, pCDM8, pSVK3, pMSG, pSVL, pBK-CMV, pBK-RSV, EBV vector, pRS, pcDNA3.1, pSecTag (A, B, C) and pYES2. By similar procedures to those mentioned above, a DNA encoding the aforementioned polypeptide is incorporated into such an expression vector, an eukaryotic host cell is transformed with the vector, and then the thus obtained transformed cell is cultured, so that the polypeptide encoded by the above DNA can be expressed in the eukaryotic host cell. When pIND/V5-His, pFLAG-CMV-2, pEGFP-N1, pEGFP-C1, or the like is used as an expression vector, the above polypeptide may be expressed as a fusion protein with a tag, such as His tag (e.g., (His)6 to (His) 10), FLAG tag, myc tag, HA tag, or GFP.

For introduction of an expression vector into a host cell, well known methods can be employed, such as electroporation, a calcium phosphate method, a liposome method, a DEAE dextran method, microinjection, viral infection, lipofection, and binding with a cell-membrane-permeable peptide.

Isolation and purification of a polypeptide of interest from host cells can be performed using known isolation techniques in combination. Examples of isolation and purification techniques include, but are not limited to, treatment using a denaturing agent such as urea or a surfactant, ultrasonication, enzymatic digestion, salting-out, solvent fractionation and precipitation, dialysis, centrifugation, ultrafiltration, gel filtration, SDS-PAGE, isoelectric focusing electrophoresis, ion exchange chromatography, hydrophobic chromatography, affinity chromatography, and reverse phase chromatography.

<Structure of antibody>

In general, antibodies are heteromultimeric glycoproteins each comprising at least two heavy chains and two light chains. Meanwhile, another class of antibodies except for IgM are heterotetrameric glycoproteins (approximately 150 kDa) each comprising two identical light (L) chains and two identical heavy (H) chains. Typically, each light chain is connected to a heavy chain via a single covalent disulfide bond. However, the number of disulfide bonds between heavy chains varies among different immunoglobulin isotypes. Each of heavy chain and light chain also has an intrachain disulfide bond(s). Each heavy chain has a variable domain (VH region) at one end thereof, to which some constant regions are bound in series. Each light chain has a variable domain (VL region) at one end thereof and has a single constant region at the opposite end thereof. The constant region of a light chain is aligned with the first constant region of a heavy chain and the light-chain variable domain is aligned with the heavy-chain variable domain. A specific region of an antibody variable domain, which is called "complementarity determining region (CDR)," exhibits specific variability so as to impart binding specificity to an antibody. A relatively conserved portion in a variable region is called a "framework region (FR)." A complete heavy-chain or light-chain variable domain comprises 4 FRs connected to each other via 3 CDRs. Such CDRs are called "CDRH1," "CDRH2," and "CDRH3," respectively, in such order from the N-terminus in a heavy chain. Similarly, for a light chain, they are called "CDRL1," "CDRL2," and "CDRL3," respectively. CDRH3 plays the most important role in terms of antibody-antigen binding specificity. In addition, CDRs in each chain are retained by FR regions in the state that they are close to each other, and they contribute to the formation of an antigen binding site of an antibody together with CDRs in a corresponding chain. Constant regions do not directly contribute to antibody-antigen binding. However, they exhibit various effector functions such as involvement in antibody-dependent cytotoxicity (ADCC activity), phagocytosis through binding to an Fcγ receptor, half-life/clearance rate via a neonatal Fc receptor (FcRn), and complement-dependent cytotoxicity (CDC activity) via a C1q component in the complement cascade.

<Antibody production>

The term "anti-MRAP2 antibody" used in the present invention refers to an antibody having an immunological reactivity with a full-length MRAP2 protein or a fragment thereof described above.

The term "immunological reactivity" used herein indicates the characteristics of an antibody binding *in vivo* or *in vitro* to an MRAP2 antigen. The tumor- or tumor cell-damaging function (e.g., death, inhibition, or regression) can be exerted via such binding. Specifically, any type of antibody may be used in the present invention as long as the antibody can bind to an MRAP2 protein to damage a tumor, preferably a cancer expressing (or having) the MRAP2 protein on a cell surface, such as leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, or perianal adenocarcinoma.

Examples of such antibodies include monoclonal antibodies, polyclonal antibodies, synthetic antibodies, multispecific antibodies (e.g., bispecific antibodies), human antibodies, humanized antibodies, chimeric antibodies, and single-chain antibodies. Examples of such antibodies also include antibody fragments (e.g., antigen binding fragments such as Fab and $F(ab')_2$). In addition, antibodies may be any class of immunoglobulin molecules such as IgG, IgE, IgM, IgA, IgD, and IgY, or any subclass thereof such as IgG1, IgG2, IgG3, IgG4, IgA1, and IgA2.

Antibodies may be further modified via acetylation, formylation, amidation, phosphorylation, or pegylation (PEG), in addition to glycosylation.

Production examples for a variety of antibodies are described below.

The polyclonal antibodies that can be used in the present invention can be obtained in a manner described below.

Serum is obtained by immunizing small animals such as mice, human antibody-producing mice, or rabbits with a naturally occurring MRAP2 protein, a recombinant MRAP2 protein that has been expressed as a protein fused with GST or the like in a microorganism such as *Escherichia coli,* or a partial peptide thereof. The serum is purified via ammonium sulfate precipitation, protein A/protein G column chromatography, DEAE ion-exchange chromatography, affinity column chromatography with a column to which an MRAP2 protein or a synthetic peptide is coupled, or the like, for preparation of polyclonal antibodies. In the Examples described below, a mouse polyclonal antibody against a domain expressed on cancer cell surfaces in an MRAP2 protein amino acid sequence was produced, and antitumor effects thereof were confirmed.

Other examples of the antibodies that can be used in the present invention include monoclonal antibodies. For example, monoclonal antibodies can be obtained in a manner described below. For example, cells expressing the MRAP2 protein on their surfaces (such as a leukemia cell line K562 or a malignant lymphoma cell line Namalwa) are administered to mice for immunization, followed by extraction of spleens from the mice. Cells are separated from each spleen and then are fused with mouse myeloma cells. Clones capable of producing an antibody having cancer cell growth inhibition action are selected from the obtained fusion cells (hybridomas). A monoclonal antibody-producing hybridoma having cancer cell growth inhibition action is isolated and cultured. An antibody of interest can be prepared via purification from the culture supernatant by a general affinity purification method.

Also, a monoclonal antibody-producing hybridoma can be produced in a manner described below, for example. First, an animal is immunized with a sensitizing antigen by a known method. In a general method, immunization is carried out by intraperitoneally or subcutaneously injecting a sensitizing antigen into a mammal. Specifically, a sensitizing antigen is diluted to an appropriate resultant amount with and suspended in PBS (Phosphate-Buffered Saline), physiological saline, or the like. If desired, an appropriate amount of a conventional adjuvant (e.g., Freund's complete adjuvant) is mixed therewith. After emulsification takes place, the resultant is administered to a mammal several times every 4 to 21 days. In addition, an adequate carrier can be used for immunization with a sensitizing antigen.

As described above, after immunization of a mammal and confirmation of an increase to a desired antibody level in serum, immunocytes are collected from the mammal and subjected to cell fusion. Particularly preferable examples of immunocytes are splenocytes.

Mammalian myeloma cells are used as relevant parent cells subjected to fusion with the above immunocytes. As the myeloma cells, the following various examples of known cell lines are preferably used: P3U1 (P3-X63Ag8U1), P3 (P3x63Ag8.653) (J. Immunol. (1979) 123, 1548-1550), P3x63Ag8U.1 (Current Topics in Microbiology and Immunology (1978) 81, 1-7), NS-1 (Kohler. G. and Milstein, C. Eur. J. Immunol. (1976). 6, 511-519), MPC-11 (Margulies. D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S. J. Exp. Med. (1978) 148, 313-323), and R210 (Galfre, G. et al., Nature (1979) 277, 131-133).

Basically, the cell fusion of immunocytes and myeloma cells described above can be carried out according to a known method such as the method of Kohler and Milstein et al. (Kohler, G. and Milstein, C. Methods Enzymol. (1981) 73, 3-46).

More specifically, the cell fusion described above is carried out, for example, in the presence of a cell fusion promoter in a conventional nutrients-containing culture solution. Examples of a fusion promoter to be used include polyethylene glycol (PEG) and Sendai virus (HVJ: hemagglutinating virus of Japan). If desired, an auxiliary agent such as dimethylsulfoxide may be further added for improvement of fusion efficiency.

The proportion of immunocytes used relative to myeloma cells used can be arbitrarily determined. For example, the number of immunocytes used is preferably one to ten times the number of myeloma cells. Examples of a culture solution that can be used for the cell fusion described above include an RPMI1640 culture solution and an MEM culture solution adequate for growth of the above myeloma cell lines as well as other conventional culture solutions used for this kind of cell culture. Further, a serum replacement solution such as fetal calf serum (FCS) can be used in combination therewith.

For cell fusion, the above immunocytes and myeloma cells are sufficiently mixed at predetermined amounts in the culture solution. A PEG solution (e.g., average molecular weight: approximately 1000 to 6000) that has been previously heated to approximately 37°C is added thereto at a concentration of generally 30% to 60% (w/v), followed by mixing. This results in formation of hybridomas of interest. Subsequently, sequential addition of an appropriate culture solution and removal of the supernatant via centrifugation are repeatedly carried out to remove cell fusion agent(s) and the like that are not preferable for the growth of hybridomas.

The thus obtained hybridomas are cultured in a conventional selection culture solution such as an HAT culture solution (a culture solution comprising hypoxanthine, aminopterin, and thymidine) for selection. Culture in such an HAT culture solution is continuously carried out for a sufficient time period (generally several days to several weeks) for death of cells (non-fused cells) other than hybridomas of interest. Next, a conventional limiting dilution method is employed to screen for hybridomas producing antibodies of interest and to carry out single cloning.

Further, as well as obtaining the above hybridomas via immunization of non-human animals with antigens, it is also possible to obtain hybridomas that produce human antibodies having a desired activity (e.g., cell growth inhibition activity) by sensitizing human lymphocytes (e.g., human lymphocytes infected with EB virus) *in vitro* with a protein, protein-expressing cells, or a lysate thereof and fusing the sensitized lymphocytes with human-derived myeloma cells having the ability to permanently divide, e.g., U266 (accession no. TIB196).

Monoclonal antibody-producing hybridomas produced as above can be passaged in a conventional culture solution. In addition, they can be preserved in liquid nitrogen for a long period of time.

Specifically, immunization is carried out using a desired antigen or cells expressing a desired antigen as sensitizing antigen(s) according to a conventional immunization method. The obtained immunocytes are fused with known parent cells by a conventional cell fusion method. Then, monoclonal antibody-producing cells (hybridomas) are screened by a conventional screening method. Thus, antibody production can be carried out.

A known human antibody-producing mouse used herein is, for example, a KM Mouse (Kirin Pharma/Medarex) or a XenoMouse (Amgen) (e.g., WO02/43478 and WO02/092812). When such mice are immunized with MRAP2 proteins or fragments thereof, complete human polyclonal antibodies can be obtained from blood. In addition, complete human monoclonal antibodies can be produced by a method of fusing splenocytes collected from immunized mice with myeloma cells.

Antigen preparation can be carried out in accordance with a method such as a method using animal cells (JP Patent Publication (Kohyo) No. 2007-530068A) or a method using a baculovirus (e.g., WO98/46777). If the immunogenicity of an antigen is low, an antigen bound to a macromolecule having immunogenicity, such as albumin, can be used for immunization.

Further, it is possible to use a genetically engineered antibody produced by cloning an antibody gene from a hybridoma, incorporating the clone into an adequate vector, introducing the vector into a host, and allowing the host to produce the antibody using a genetic engineering techniques (see, for example, Carl, A. K. Borrebaeck, James, W. Larrick, THERAPEUTIC MONOCLONAL ANTIBODIES, Published in the United Kingdom by MACMILLAN PUBLISHERS LTD, 1990). Specifically, cDNA of a variable region (V region) of an antibody is synthesized from mRNA of a hybridoma with a reverse transcriptase. After DNA encoding a V region of an antibody of interest is obtained, such DNA is ligated to desired DNA encoding an antibody constant region (C region). The resultant is incorporated into an expression vector. Alternatively, DNA encoding an antibody V region may be incorporated into an expression vector comprising DNA of an antibody C region. Such DNA is incorporated into an expression vector in a manner such that it is expressed under control of an expression control region such as an enhancer or a promoter. Next, host cells are transformed with such expression vector, thereby allowing the antibody to be expressed.

Monoclonal antibodies include human monoclonal antibodies and non-human animal monoclonal antibodies (e.g., mouse monoclonal antibodies, rat monoclonal antibodies, rabbit monoclonal antibodies, and chicken monoclonal antibodies). Monoclonal antibodies can be produced by culturing hybridomas obtained via fusion of myeloma cells and splenocytes from non-human mammals (e.g., mice or human antibody-producing mice) immunized with MRAP2 proteins or fragments thereof.

A chimeric antibody is an antibody produced by combining sequences from different animals. An example thereof is an antibody consisting of mouse antibody heavy-chain and light-chain variable regions and human antibody heavy-chain and light-chain constant regions. Such a chimeric antibody can be produced by a known method. For example, a chimeric antibody can be obtained by ligating DNA encoding an antibody V region to DNA encoding a human antibody C region, incorporating the resultant into an expression vector, introducing the vector into a host and allowing the host to produce an antibody.

Polyclonal antibodies include antibodies obtained by immunizing human antibody-producing animals (e.g., mice) with MRAP2 proteins or fragments thereof.

A humanized antibody is an engineered antibody, and it is sometimes referred to as a "reshaped human antibody." A humanized antibody is constructed by transplanting CDRs of an immunized animal-derived antibody into complementarity determining regions of a human antibody. Also, general genetic engineering techniques therefor are known.

Specifically, a DNA sequence designed to ligate mouse antibody CDRs to framework regions (FRs) of a human antibody is synthesized by PCR method using several oligonucleotides prepared to have portions overlapping each other at their ends. A humanized antibody can be obtained by ligating the above obtained DNA to DNA encoding a human antibody constant region, incorporating the resultant into an expression vector, introducing the vector into a host and allowing the host to produce an antibody (see EP-A-239400 and WO96/02576). Human antibody FRs to be ligated to each other via CDRs are selected, provided that complementarity determining regions can form a good antigen binding site. If necessary, amino acids in framework regions of an antibody variable region may be substituted in such a manner that complementarity determining regions in a reshaped human antibody form an appropriate antigen binding site (Sato K. et al., Cancer Research 1993, 53: 851-856). In addition, the framework regions may be substituted with framework regions from various human antibodies (see WO99/51743).

After a chimeric antibody or a humanized antibody is produced, amino acids in a variable region (e.g., FR) or a constant region may be, for example, substituted with different amino acids.

Here, the amino acid substitution is a substitution of, for example, less than 15, less than 10, not more than 8, not more than 7, not more than 6, not more than 5, not more than 4, not more than 3, or not more than 2 amino acids, preferably 1 to 5 amino acids, and more preferably 1 or 2 amino acids. A substituted antibody should be functionally equivalent to an unsubstituted antibody. The substitution is preferably a conservative amino acid substitution, which is a substitution between amino acids having similar characteristics in terms of charge, side chains, polarity, aromaticity, and the like. For example, amino acids having similar characteristics can be classified into the following types: basic amino acids (arginine, lysine, and histidine); acidic amino acids (aspartic acid and glutamic acid); uncharged polar amino acids (glycine, asparagine, glutamine, serine, threonine, cysteine, and tyrosine); nonpolar amino acids (leucine, isoleucine, alanine, valine, proline, phenylalanine, tryptophan, and methionine); branched-chain amino acids (threonine, valine, isoleucine); and aromatic amino acids (phenylalanine, tyrosine, tryptophan, and histidine).

Antibodies of the present invention may be modified antibodies. An example of a modified antibody is an antibody bound to a molecule such as polyethylene glycol (PEG). Regarding modified antibody of the present invention, substances that bind to an antibody are not limited. Such a modified antibody can be obtained by chemically modifying an obtained antibody. A method of such modification has been already established in the field related to the present invention.

The expression "functionally equivalent" used herein indicates a situation in which an antibody of interest has biological or biochemical activity similar to that of an antibody of the present invention. Specifically, such antibody has a function of damaging tumors and causes essentially no rejection reaction when applied to humans. An example of such activity is cell growth inhibition activity or binding activity.

A known method for preparing a polypeptide functionally equivalent to a given polypeptide that is well known to persons skilled in the art is a method comprising introducing a mutation into the polypeptide. For instance, a person skilled in the art can adequately introduce a mutation into an antibody of the present invention using a site-specific mutagenesis method (Hashimoto-Gotoh, T. et al., (1995) Gene 152, 271-275; Zoller, MJ., and Smith, M. (1983) Methods Enzymol. 100, 468-500; Kramer, W. et al., (1984) Nucleic Acids Res. 12, 9441-9456; Kramer, W. and Fritz, HJ., (1987) Methods Enzymol. 154, 350-367; Kunkel, TA., (1985) Proc. Natl. Acad. Sci. USA. 82, 488-492; or Kunkel (1988) Methods Enzymol. 85, 2763-2766) or the like. Thus, an antibody functionally equivalent to the antibody of the present invention can be prepared.

An antibody capable of recognizing an epitope of an MRAP2 protein recognized by the aforementioned anti-MRAP2 antibody can be obtained by a method known to persons skilled in the art. For example, it can be obtained by: a method comprising determining an epitope of an MRAP2 protein recognized by the anti-MRAP2 antibody by a general method (e.g., epitope mapping) and producing an antibody using a polypeptide having an amino acid sequence contained in the epitope as an immunogen; or a method comprising determining an epitope of a produced antibody by a general method and selecting an antibody having an epitope identical to an epitope of the anti-MRAP2 antibody. Here, the term "epitope" refers to a polypeptide fragment having antigenicity or immunogenicity in mammals and preferably in humans. The smallest unit thereof consists of approximately 7 to 12 amino acids and preferably 8 to 11 amino acids.

The affinity constant Ka (kon/koff) of an antibody of the present invention is preferably at least $10^7$ $M^{-1}$, at least $10^8$ $M^{-1}$, at least $5 \times 10^8$ $M^{-1}$, at least $10^9$ $M^{-1}$, at least $5 \times 10^9$ $M^{-1}$, at least $10^{10}$ $M^{-1}$, at least $5 \times 10^{10}$ $M^{-1}$, at least $10^{11}$ $M^{-1}$, at least $5 \times 10^{11}$ $M^{-1}$, at least $10^{12}$ $M^{-1}$, or at least $10^{13}$ $M^{-1}$.

An antibody of the present invention can be conjugated with an antitumor agent. Binding between an antibody and an antitumor agent can be carried out via a spacer having a group reactive to an amino group, a carboxyl group, a hydroxy group, a thiol group, or the like (e.g., an imidyl succinate group, a formyl group, a 2-pyridyldithio group, a maleimidyl group, an alkoxycarbonyl group, or a hydroxy group).

Examples of antitumor agents include the following antitumor agents known in references or the like: paclitaxel, doxorubicin, daunorubicin, cyclophosphamide, methotrexate, 5-fluorouracil, thiotepa, busulfan, improsulfan, piposulfan, benzodopa, carboquone, meturedopa, uredopa, altretamine, triethylenemelamine, triethylenephosphoramide, triethilenethiophosphoramide, trimethylolomelamine, bullatacin, bullatacinone, camptothecin, bryostatin, callystatin, cryptophycin 1, cryptophycin 8, dolastatin, duocarmycin, eleutherobin, pancratistatin, sarcodictyin, spongistatin, chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, calicheamicin, dynemicin, clodronate, esperamicin, aclacinomycin, actinomycin, authramycin, azaserine, bleomycin, cactinomycin, carabicin, carminomycin, carzinophilin, chromomycin, dactinomycin, detorbicin, 6-diazo-5-oxo-L-norleucine, ADRIAMYCIN, epirubicin, esorubicin, idarubicin, marcellomycin, mitomycin C, mycophenolic acid, nogalamycin, olivomycin, peplomycin, potfiromycin, puromycin, quelamycin, rodorubicin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine, androgens such as calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, epothilone, etoglucid, lentinan, lonidamine, maytansine, ansamitocine, mitoguazone, mitoxantrone, mopidanmol, nitraerine, pentostatin, phenamet, pirarubicin, losoxantrone, podophyllinic acid, 2-ethylhydrazide, procarbazine, razoxane, rhizoxin, schizophyllan, spirogermanium, tenuazonic acid, triaziquone, roridine A, anguidine, urethane, vindesine, dacarbazine, mannomustine, mitobronitol, mitolactol, pipobroman, gacytosine, docetaxel, chlorambucil, gemcitabine, 6-thioguanine, mercaptopurine, cisplatin, oxaliplatin, carboplatin, vinblastine, etoposide, ifosfamide, mitoxantrone, vincristine, vinorelbine, novantrone, teniposide, edatrexate, daunomycin, aminopterin, xeloda, ibandronate, irinotecan, topoisomerase inhibitor, difluoromethylornithine (DMFO), retinoic acid, capecitabine, and pharmacologically acceptable salts or derivatives thereof.

Alternatively, it is also possible to bind a radioactive isotope such as $^{211}At$, $^{131}I$, $^{125}I$, $^{90}Y$, $^{186}Re$, $^{188}Re$, $^{153}Sm$, $^{212}Bi$, $^{32}P$, $^{175}Lu$, or $^{176}Lu$ known in references and the like to an antibody of the present invention. It is desirable for such radioactive isotopes to be effective for tumor treatment or diagnosis.

An antibody of the present invention is preferably an antibody having an immunological reactivity with MRAP2 or an antibody capable of specifically recognizing MRAP2. Such an antibody should be an antibody having a structure that allows a subject animal to which the antibody is administered to completely or almost completely avoid a rejection reaction. If the subject animal is a human, examples of such antibodies include human antibodies, humanized antibodies, chimeric antibodies (e.g., human-mouse chimeric antibodies), single-chain antibodies, and bispecific antibodies. Such an antibody is a recombinant antibody having human antibody-derived heavy-chain and light-chain variable regions, a recombinant antibody having heavy-chain and light-chain variable regions each consisting of non-human animal antibody-derived complementarity determining regions (CDR1, CDR2, and CDR3) and human antibody-derived framework regions, or a recombinant antibody having non-human animal antibody-derived heavy-chain and light-chain variable regions and human antibody-derived heavy-chain and light-chain constant regions. The first two antibodies are preferable.

The above recombinant antibody can be produced in the manner described below. DNA encoding a monoclonal antibody against human MRAP2 (e.g., a human monoclonal antibody, a mouse monoclonal antibody, a rat monoclonal antibody, a rabbit monoclonal antibody, or a chicken monoclonal antibody) is cloned from an antibody-producing cell such as a hybridoma. DNAs encoding a light-chain variable region and a heavy-chain variable region of the antibody are produced by an RT-PCR method or the like using the obtained clone as a template. Then, the sequences of a light-chain variable region and a heavy-chain variable region or the sequences of CDR1, CDR2, and CDR3 are determined by the Kabat EU numbering system (Kabat et al., Sequences of Proteins of Immunological Interest, 5th Ed. Public Health Service, National Institute of Health, Bethesda, Md. (1991)).

Further, such DNAs encoding variable regions or DNAs encoding CDRs are produced by genetic engineering techniques (Sambrook et al., Molecular Cloning A Laboratory Manual, Cold Spring Harbor Laboratory Press (1989)) or a DNA synthesizer. Here, the above human monoclonal antibody-producing hybridoma can be produced by immunizing a human antibody-producing animal (e.g., a mouse) with human MRAP2 and fusing splenocytes removed from the animal with myeloma cells. In addition to the above, if necessary, DNAs encoding human antibody-derived light-chain or heavy-chain variable regions and constant regions are produced by genetic engineering techniques or a DNA synthesizer.

In the case of a humanized antibody, DNA in which the CDR coding sequences in a DNA encoding a human antibody-derived light-chain or heavy-chain variable region have been substituted with corresponding CDR coding sequences of an antibody from a non-human animal (e.g., a mouse, a rat, or a chicken) is produced. The DNA obtained as above is ligated to the DNA encoding a constant region of a human antibody-derived light chain or heavy chain. Thus, DNA encoding a humanized antibody can be produced.

In the case of a chimeric antibody, DNA encoding an antibody light-chain or heavy-chain variable region from a non-human animal (e.g., a mouse, a rat, or a chicken) is ligated to the DNA encoding a human antibody-derived light-chain or heavy-chain constant region. Thus, DNA encoding a chimeric antibody can be produced.

A single-chain antibody is an antibody in which a heavy-chain variable region and a light-chain variable region are linearly ligated to each other via a linker. DNA encoding a single-chain antibody can be produced by ligating DNA encoding a heavy-chain variable region, DNA encoding a linker, and a DNA encoding a light-chain variable region together. Here, a heavy-chain variable region and a light-chain variable region are those from a human antibody or those from a human antibody in which CDRs alone have been substituted with CDRs of an antibody from a non-human animal (e.g., a mouse, a rat, or a chicken). In addition, the linker consists of 12 to 19 amino acids. An example thereof is (G4S)3 consisting of 15 amino acids (G. -B. Kim et al., Protein Engineering Design and Selection 2007, 20 (9): 425-432).

A bispecific antibody (diabody) is an antibody capable of specifically binding to two different epitopes. DNA encoding a bispecific antibody can be produced by, for example, ligating DNA encoding a heavy-chain variable region A, DNA encoding a light-chain variable region B, DNA encoding a heavy-chain variable region B, and DNA encoding a light-chain variable region A together in such order (provided that DNA encoding a light-chain variable region B and DNA encoding a heavy-chain variable region B are ligated to each other via DNA encoding a linker described above).. Here, both a heavy-chain variable region and a light-chain variable region are those from a human antibody or those from a human antibody in which CDRs alone have been substituted with CDRs of an antibody from a non-human animal (e.g., a mouse, a rat, or a chicken).

Recombinant DNA produced as above is incorporated into one or a plurality of appropriate vector(s). Each such vector is introduced into a host cell (e.g., a mammal cell, a yeast cell, or an insect cell) for (co)expression. Thus, a recombinant antibody can be produced. See, P. J. Delves., ANTIBODY PRODUCTION ESSENTIAL TECHNIQUES., 1997 WILEY, P. Shepherd and C. Dean., Monoclonal Antibodies., 2000 OXFORD UNIVERSITY PRESS; J. W. Goding, Monoclonal Antibodies: Principles and Practice., 1993 ACADEMIC PRESS.

The above antibodies preferably have cytotoxic activity, thereby exhibiting antitumor effects.

In addition, a hybridoma capable of producing a different human antibody or a non-human animal antibody (e.g., a mouse antibody) against human MRAP2 is produced. A monoclonal antibody produced by the hybridoma is collected. Then, it is determined whether or not the obtained antibody is an antibody of interest using, as indicators, immunological binding activity to human MRAP2 and cytotoxic activity. Thus, a monoclonal antibody-producing hybridoma of interest is identified. Thereafter, as described above, DNAs encoding heavy-chain and light-chain variable regions of an antibody of interest are produced from the hybridoma and sequenced. The DNAs are used for production of different antibodies.

Further, the above antibody of the present invention may have a substitution, deletion, or addition of one or several (and preferably, 1 or 2) amino acid(s), particularly in a framework region sequence and/or a constant region sequence, as long as it has the specificity of specifically recognizing MRAP2. Here, the term "several amino acids" indicates 2 to 5 and preferably 2 or 3 amino acids.

Furthermore, according to the present invention, DNA encoding the above antibody of the present invention, DNA encoding a heavy chain or light chain of the antibody, or DNA encoding a heavy-chain or light-chain variable region of the antibody is also provided.

Complementarity determining regions (CDRs) encoded by DNAs of the above sequences are regions that determine antibody specificity. Therefore, sequences encoding the other regions (i.e., constant regions and framework regions) in an antibody may be sequences from a different antibody. Here, different antibodies include antibodies from non-human organisms. However, in view of reduction of side effects, human-derived antibodies are preferable. That is to say, in the above DNA, regions encoding framework regions and constant regions of heavy and light chains preferably comprise nucleotide sequences encoding the relevant amino acid sequences from a human antibody.

DNA of the present invention can be obtained by, for example, the aforementioned methods or the following methods. First, total RNA is prepared from a hybridoma associated with an antibody of the present invention using a commercially available RNA extraction kit. Then, cDNA is synthesized with a reverse transcriptase using random primers or the like. Next, cDNA encoding an antibody is amplified by a PCR method using, as primers, oligonucleotides having sequences conserved in variable regions of known mouse antibody heavy-chain and light-chain genes. Sequences encoding constant regions can be obtained by amplifying known sequences by a PCR method. The nucleotide sequence of the DNA can be determined by a general method involving, for example, incorporation into a plasmid or phage for sequence determination.

It is thought that antitumor effects of an anti-MRAP2 antibody used in the present invention upon MRAP2-expressing cancer cells are exhibited through effector cell-mediated antibody-dependent cellular cytotoxicity (ADCC) activity against MRAP2-expressing cells or complement-dependent cytotoxicity (CDC) activity against MRAP2-expressing cells.

Accordingly, the activity of an anti-MRAP2 antibody used in the present invention can be evaluated via *in vitro* determination of ADCC activity or CDC activity to MRAP2-expressing cancer cells as specifically described in the Examples mentioned below.

An anti-MRAP2 antibody used in the present invention binds to an MRAP2-protein on a cancer cell and exhibits antitumor effects based on the above activity. Therefore, such antibody is believed to be useful for cancer treatment or prevention. Specifically, according to the present invention, a pharmaceutical composition for treatment and/or prevention of cancer that comprises, as an active ingredient, an anti-MRAP2 antibody, is provided. When an anti-MRAP2 antibody is used for the purpose of administering the antibody to humans (antibody treatment), it is preferably used in the form of a human antibody or a humanized antibody in order to reduce immunogenicity.

In addition, as the binding affinity between an anti-MRAP2 antibody and an MRAP2 protein on a cancer cell surface becomes higher, stronger antitumor activity can be exhibited by an anti-MRAP2 antibody. Therefore, if an anti-MRAP2 antibody having high binding affinity to an MRAP2 protein can be obtained, even stronger antitumor effects can be expected to be exhibited. Accordingly, it becomes possible to use such antibody as a pharmaceutical composition for treatment and/or prevention of cancer. As described above, for high binding affinity, the affinity constant Ka (kon/koff) is preferably at least $10^7$ $M^{-1}$, at least $10^8$ $M^{-1}$, at least $5 \times 10^8$ $M^{-1}$, at least $10^9$ $M^{-1}$, at least $5 \times 10^9$ $M^{-1}$, at least $10^{10}$ $M^{-1}$, at least $5 \times 10^{10}$ $M^{-1}$, at least $10^{11}$ $M^{-1}$, at least $5 \times 10^{11}$ $M^{-1}$, at least $10^{12}$ $M^{-1}$, or at least $10^{13}$ $M^{-1}$.

<Binding to antigen expression cells>

The capacity of an antibody to bind to MRAP2 can be determined via binding assay using, for example, ELISA, a Western blot method, immunofluorescence, or flowcytometry analysis as described in the Examples.

<Immunohistochemical staining>

An antibody that recognizes MRAP2 can be tested in terms of reactivity with MRAP2 by an immunohistochemical method well-known to persons skilled in the art using a frozen tissue section fixed with paraformaldehyde or acetone or a paraffin-embedded tissue section fixed with paraformaldehyde. Such section is prepared from a tissue obtained from a patient during surgery, a bone marrow tissue, lymph node, peripheral blood cells, or bone marrow cells of a patient, or a tissue obtained from an animal carrying xenograft tissue that has been inoculated with a cell line that expresses MRAP2 naturally or after transfection thereof.

For immunohistochemical staining, an antibody immunologically reactive to MRAP2 can be stained by a variety of methods. For example, it can be visualized by reacting with a horseradish peroxidase-conjugated goat anti-mouse antibody or goat anti-rabbit antibody.

<Pharmaceutical composition>

The present invention provides a pharmaceutical composition (or medicament) comprising an antibody of the present invention, i.e., an antibody against MRAP2 or fragment (preferably antigen binding fragment) thereof described above. The pharmaceutical composition (or medicament) of the present invention usually comprises an effective amount of the antibody against MRAP2 or fragment (preferably antigen binding fragment) thereof described above.

A target of the pharmaceutical composition for treatment and/or prevention of a cancer of the present invention is not particularly limited as long as the target is a cancer (cell) expressing the MRAP2 gene (usually, on a cell surface).

Both the terms "tumor" and "cancer" used herein refer to malignant neoplasm, and thus they are used in an exchangeable manner.

A cancer that can be a target in the present invention is a cancer expressing a gene encoding a polypeptide comprising the amino acid sequence of SEQ ID NO: 2, 4, 6, or 8 or a partial sequence consisting of 7 or more consecutive amino acids of said amino acid sequence, and is preferably a cancer expressing such polypeptide on a cell surface. The cancer that can be a target in the present invention is preferably leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, or perianal adenocarcinoma. Specific examples of these cancers include, but are not limited to, acute non-lymphocytic leukemia, chronic lymphocytic leukemia, acute granulocytic leukemia, chronic granulocytic leukemia, acute promyelocytic leukemia, adult T-cell leukemia, aleukemic leukemia, leukocythemic leukemia, basophilic leukemia, blastic leukemia, bovine leukemia, chronic myeloleukemia, leukemia cutis, embryonal leukemia, eosinophilic leukemia, Gross leukemia, Rieder cell leukemia, Schilling's leukemia, stem cell leukemia, subleukemic leukemia, undifferentiated cell leukemia, hairy cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphotropic leukemia, lymphoid leukemia, lymphosarcoma cell leukemia, mast cell leukemia, megakaryocytic leukemia, micromyeloblastic leukemia, monocytic leukemia, myeloblastic leukemia, myeloleukemia, myeloid granulocytic leukemia, myelomonocytic leukemia, Naegeli leukemia, plasma cell leukemia, plasmacytic leukemia, promyelocytic leukemia, non-Hodgkin lymphoma (Burkitt lymphoma (BL), small lymphocytic lymphoma/chronic lymphocytic leukemia (SLL/CLL), mantle cell lymphoma (MCL), follicular lymphoma (FL), diffuse large B-cell lymphoma (DLCL), marginal zone lymphoma (MZL), hairy cell leukemia (HCL), lymphoplasmacytic leukemia (LPL), extranodal marginal zone B cell lymphoma of mucosa-associated lymphoid tissue (MALT), mediastinal large cell lymphoma, intravascular large cell lymphoma, primary effusion lymphoma, precursor B-cell lymphoblastic leukemia/lymphoma, precursor T-cell and NK-cell lymphoma (precursor T-cell lymphoblastic lymphoma, NK-cell lymphoblastic lymphoma), mature T- and NK-cell neoplasms (including peripheral T-cell lymphoma and leukemia (PTL)), adult T-cell leukemia/T-cell lymphoma and large granular lymphocytic leukemia, chronic T-cell lymphocytic leukemia/prolymphocytic leukemia, T-cell large granular lymphocytic leukemia, aggressive NK-cell leukemia, extranodal T-/NK-cell lymphoma, enteropathy-type T-cell lymphoma, hepatosplenic T-cell lymphoma, anaplastic large cell lymphoma (ALCL), angiocentric and angioimmunoblastic T-cell lymphoma, mycosis fungoides/Sezary's syndrome, cutaneous T-cell lymphoma (CTCL)), Hodgkin lymphoma, non-small cell lung cancer, squamous cell carcinoma (epidermoid cancer), lung adenocarcinoma, large cell lung cancer, small cell lung cancer, glioma, astrocytoma, brainstem glioma, ependymoma, oligodendroglioma, nonglial tumor, acoustic neurilemoma, craniopharyngioma, medulloblastoma, meningioma, pineocytoma, pineoblastoma, primary brain lymphoma, germ cell tumor, superficial colorectal cancer, protuberant colorectal cancer, ulcerative infiltrative colorectal cancer, diffuse infiltrative colorectal cancer, basal cell cancer, prickle cell cancer, melanoma, superficial spreading melanoma, nodular melanoma, malignant lentigo melanoma, acral lentiginous melanoma, neuroblastoma, ganglioneuroblastoma, ganglioma, insulinoma, gastrinoma, glucagonoma, VIPoma, somatostatin-secreting tumor, carcinoid, islet cell tumor, protuberant gastric cancer, ulcerative localized gastric cancer, ulcerative infiltrative gastric cancer, diffuse infiltrative gastric cancer, hepatocellular cancer and hepatoblastoma, epithelial ovarian cancer, borderline tumor, germ cell tumor, stromal tumor, serous adenocarcinoma, clear cell adenocarcinoma, endometrioid adenocarcinoma, transitional cell cancer, mucous adenocarcinoma, mixed ovary cancer, squamous cell carcinoma, esophageal adenocarcinoma, renal cell cancer, kidney adenocarcinoma, hypernephroma, renal fibrosarcoma, transitional cell cancer (renal pelvis and/or uterer), mastocytoma, perianal adenoma, and perianal adenocarcinoma.

In addition, the subject animal of the present invention is a mammal. Examples thereof include mammals such as primates, pet animals, livestock animals, sport animals, and experimental animals. Humans, dogs, and cats are particularly preferable.

When an antibody used in the present invention is used in a pharmaceutical composition, it can be formulated by a method known to persons skilled in the art. For instance, it can be parenterally used in the form of an injection being an aseptic solution or suspension liquid in water or other pharmacologically acceptable solution. For example, in one possible case, it can be formulated with the combined use of a pharmacologically acceptable carrier or medium or additive and specifically sterilized water, physiological saline, plant oil, an emulsifier, a suspending agent, a surfactant, a stabilizer, a flavoring agent, an excipient, a vehicle, a preservative, a binder or the like in an appropriate manner by mixing in a unit dosage form required for a generally acceptable pharmaceutical formulation. The amount of an active ingredient in a formulation is determined such that an appropriate dosage within the indicated range can be achieved.

An aseptic composition for injection purposes can be formulated in accordance with general formulation practice using a vehicle such as distilled water for injection purposes.

Examples of an aqueous solution for injection purposes include physiological saline and isotonic solutions comprising glucose and other auxiliary agents, such as D-sorbitol, D-mannose, D-mannitol, and sodium chloride. Such solution may be used together with an appropriate solubilizing agent, for example, alcohols such as ethanol and polyalcohol (e.g., propylene glycol, polyethylene glycol), and nonion surfactants such as polysorbate 80™ and HCO-60.

Examples of oil liquid include sesame oil and soybean oil. Such oil liquid may be used in combination with benzyl benzoate or benzyl alcohol as a solubilizing agent. In addition, it may be mixed with a buffering agent such as a phosphate buffer solution, a sodium acetate buffer solution, a soothing agent such as procaine hydrochloride, a stabilizer such as benzyl alcohol, phenol, and/or an antioxidant. In general, a formulated injection solution is introduced into an adequate ample.

The administration is performed orally or parenterally, and preferably parenterally. Specific examples of dosage forms include injectable dosage forms, intranasal dosage form, transpulmonary dosage form, and percutaneous dosage form. For example, injectable dosage form can be systemically or locally administered via intravenous injection, intramuscular injection, intraperitoneal injection, or subcutaneous injection. Alternatively, an antibody of the present invention may be administered directly to a tumor by local administration, such as injection, infusion, or implantation of a sustained-release formation, to the tumor.

In addition, the administration method can be appropriately determined depending on e.g., patient age, weight, gender, and/or symptoms. For example, a single dose of a pharmaceutical composition comprising the antibody or a polynucleotide encoding the antibody can be selected within a range of 0.0001 mg to 1,000 mg per kg of body weight. Alternatively, the dose can be selected, for example, within a range of 0.001 to 100,000 mg per patient's body; however, it is not necessarily limited thereto. The dose and the administration method vary depending on e.g., patient age, weight, gender, and symptoms. However, persons skilled in the art can appropriately select the dose and the method.

The cancer described above, particularly, a cancer expressing MRAP2 on a cell surface, preferably leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, or perianal adenocarcinoma can be treated and/or prevented by administering an antibody of the present invention or fragment thereof, or the pharmaceutical composition comprising the same to a subject.

Further, a method for treating and/or preventing a cancer, which comprises administering, to a subject, the pharmaceutical composition (or medicament) of the present invention in combination with an antitumor agent as listed above or a pharmaceutical composition (or medicament) comprising the antitumor agent, is also included in the present invention. A target cancer is the same as above. The antibody or fragment thereof according to the present invention and the antitumor agent can be administered concurrently or separately to the subject. In the case of separately administering them, either of the pharmaceutical compositions can be administered first or later, and their dosing intervals, doses, administration routes, and the number of doses can be appropriately selected by a specialist physician. In the case of concurrently administering them, for example, a pharmaceutical composition in a dosage form obtained by mixing the antibody or fragment thereof according to the present invention and the antitumor agent in a pharmacologically acceptable carrier (or medium) for formulation is also included in the present invention. The description about prescription, formulation, administration routes, doses, cancers, etc. regarding pharmaceutical compositions and dosage forms containing antibodies of the present invention is applicable to all of the pharmaceutical compositions and dosage forms containing antitumor agents.

Accordingly, the present invention also provides a pharmaceutical combination for treatment and/or prevention of a cancer, which comprises the pharmaceutical composition of the present invention and a pharmaceutical composition comprising an antitumor agent as listed above, and a method for treating and/or preventing a cancer, which comprises administering the same. In addition, the present invention also provides a pharmaceutical composition for treatment and/or prevention of a cancer, which comprises an antibody or fragment thereof according to the present invention and an antitumor agent together with a pharmacologically acceptable carrier and/or additive.

## Examples

The present invention is hereafter described in greater detail with reference to the following examples, although the scope of the present invention is not limited thereto.

### Example 1: Identification of new cancer antigen protein by SEREX method

#### (1) Construction of cDNA library

Total RNA was extracted from a testis tissue of a healthy dog by an Acid guanidium-Phenol-Chloroform method and then a polyA RNA was purified according to protocols included with an Oligotex-dT30 mRNA purification Kit (Takara Shuzo Co., Ltd.).

A canine testis cDNA phage library was synthesized using the thus obtained mRNA (5 μg). The cDNA phage library was constructed using a cDNA Synthesis Kit, a ZAP-cDNA Synthesis Kit, and a ZAP-cDNA GigapackIII Gold Cloning Kit (STRATAGENE) according to protocols included with the kits. The size of the thus constructed cDNA phage library was $1\times 10^6$pfu/ml.

(2) Screening of cDNA library using serum

Immunoscreening was performed using the above constructed canine testis cDNA phage library. Specifically, host *Escherichia coli* (XL1-Blue MRF') was infected with the phage on an NZY agarose plate (Φ90 × 15 mm) so as to obtain approximately 2500 clones. *E. coli* cells were cultured at 42°C for 3 to 4 hours to form plaques. The plate was covered with a nitrocellulose membrane (Hybond C Extra: GE Healthcare Bio-Science) impregnated with IPTG (isopropyl-β-D-thiogalactoside) at 37°C for 4 hours, so that the protein was induced, expressed, and then transferred to the membrane. Subsequently, the membrane was taken and then immersed in TBS (10 mM Tris-HCl, 150 mM NaCl, and pH 7.5) containing 0.5% powdered skim milk, followed by overnight shaking at 4°C, thereby suppressing nonspecific reaction. The filter was reacted with a 500-fold diluted serum of a canine patient at room temperature for 2 to 3 hours.

As the above serum of a canine patient, a serum collected from a canine patient with leukemia was used. These sera were stored at -80°C and then subjected to pre-treatment immediately before use. A method for pretreatment of serum is as follows. Specifically, host *Escherichia coli* (XL1-Blue MRF') was infected with a λ ZAP Express phage in which no foreign gene had been inserted and then cultured overnight on a NZY plate medium at 37°C. Subsequently, buffer (0.2 M $NaHCO_3$ and pH 8.3) containing 0.5 M NaCl was added to the plate, the plate was left to stand at 4°C for 15 hours, and then a supernatant was collected as an *Escherichia coli*/phage extract. Next, the thus collected *Escherichia coli*/phage extract was applied to an NHS-column (GE Healthcare Bio-Science), so that an *Escherichia coli*·phage-derived protein was immobilized. The serum of a canine patient was applied to the protein-immobilized column for reaction and then antibodies adsorbed to the *Escherichia coli* and phage were removed from the serum. The serum fraction that had passed through the column was diluted 500-fold with TBS containing 0.5% powdered skim milk. The resultant was used as an immunoscreening material.

The above membrane onto which the treated serum and the protein had been blotted was washed 4 times with TBS-T (0.05% Tween20/TBS) and then caused to react with goat anti-dog IgG (Goat anti-Dog IgG-h+L HRP conjugated (BETHYL Laboratories)) diluted 5000-fold with TBS containing 0.5% powdered skim milk as a secondary antibody for 1 hour at room temperature. Detection was performed via an enzyme coloring reaction using an NBT/BCIP reaction solution (Roche). Colonies that matched sites positive for a coloring reaction were collected from the NZY agarose plate (Φ90 × 15 mm) and then lysed in 500 μl of an SM buffer (100 mM NaCl, 10 mM $MgClSO_4$, 50 mM Tris-HCl, 0.01% gelatin, and pH 7.5). Until colonies positive for coloring reaction were unified, secondary screening and tertiary screening were repeated so that approximately 10,000 phage clones reacting with serum IgG were screened for by a method similar to the above. Thus, 1 positive clone was isolated.

(3) Homology search for isolated antigen gene

For nucleotide sequence analysis of the 1 positive clone isolated by the above method, a procedure for conversion from phage vectors to plasmid vectors was performed. Specifically, 200 μl of a solution was prepared to contain host *Escherichia coli* (XL1-Blue MRF') so that absorbance $OD_{600}$ was 1.0. The solution was mixed with 100 μl of a purified phage solution and then with 1 μl of an ExAssist helper phage (STRATAGENE), followed by 15 minutes of reaction at 37°C. Three (3) ml of LB medium was added and then culture was performed at 37°C for 2.5 to 3 hours. Immediately after culture, the temperature of the solution was kept at 70°C by water bath for 20 minutes, centrifugation was performed at 4°C and 1000 × g for 15 minutes, and then the supernatant was collected as a phagemid solution. Subsequently, 200 μl of a solution was prepared to contain phagemid host *Escherichia coli* (SOLR) so that absorbance $OD_{600}$ was 1.0. The solution was mixed with 10 μl of a purified phage solution, followed by 15 minutes of reaction at 37°C. The solution (50 μl) was seeded on LB agar medium containing ampicillin (final concentration of 50 μg/ml) and then cultured overnight at 37°C. Transformed SOLR single colony was collected and then cultured in LB medium containing ampicillin (final concentration: 50 μg/ml) at 37°C. A plasmid DNA containing the insert of interest was purified using a QIAGEN plasmid Miniprep Kit (QIAGEN).

The purified plasmid was subjected to analysis of the full-length insert sequence by a primer walking method using the T3 primer of SEQ ID NO: 25 and the T7 primer of SEQ ID NO: 26. As a result of sequence analysis, the gene sequence of SEQ ID NO: 3 was obtained. A sequence identity search program, BLAST search (http://www.ncbi.nlm.nih.gov/BLAST/), was performed using the nucleotide sequence of the gene and the amino acid sequence thereof. As a result of this sequence identity search with known genes, it was revealed that the obtained gene was MRAP2 gene. The sequence identity with human MRAP2, a human homolog of canine MRAP2, was 91% in terms of nucleotide sequence and 94% in terms of amino acid sequence. The sequence identity with feline MRAP2 was 95% in terms of nucleotide sequence and 96% in terms of amino acid sequence. The sequence identity with mouse MRAP2, a mouse homolog of canine MRAP2, was 84% in terms of nucleotide sequence and 88% in terms of amino acid sequence. The nucleotide sequence of human MRAP2 is shown in SEQ ID NO: 1 and the amino acid sequence of the same is shown in SEQ ID NO: 2. The nucleotide sequence of feline MRAP2 is shown in SEQ ID NO: 5 and the amino acid sequence of the same is shown in SEQ ID NO: 6. The nucleotide sequence of mouse MRAP2 is shown in SEQ ID NO: 7 and the amino acid sequence of the same is shown in SEQ ID NO: 8.

(4) Gene expression analysis in each tissue

Expression of the gene obtained by the above method in canine, human, and mouse various normal tissues, various tumor tissues, and various cancer cell lines was examined by an RT-PCR (reverse transcription-PCR) method. A reverse transcription reaction was performed as follows. Specifically, total RNA was extracted from each tissue (50 mg to 100 mg) and each cell line (5 to 10 $\times$ $10^6$ cells) using a TRIZOL reagent (Thermo Fisher Scientific) according to protocols included therewith. cDNA was synthesized using the total RNA and Superscript First-Strand Synthesis System for RT-PCR ((Thermo Fisher Scientific) according to protocols included with the kit. Gene Pool cDNA (Thermo Fisher Scientific), QUICK-Clone cDNA (Clontech Laboratories, Inc.), and Large-Insert cDNA Library (Clontech Laboratories, Inc.) were used as cDNAs from human normal tissues (brain, testis, colon, and placenta). PCR was performed as follows using primers specific to the obtained gene (canine primers: SEQ ID NOS: 27 and 28, human primers: SEQ ID NOS: 29 and 30, mouse primers: SEQ ID NOS: 31 and 32). Specifically, PCR was performed by repeating 30 times a cycle of 94°C/30 seconds, 55°C/30 seconds, and 72°C/1 minute using a Thermal Cycler (BIO RAD) and a reaction solution adjusted to a total amount of 25 $\mu$l through addition of each reagent and an attached buffer (0.25 $\mu$l of the cDNA sample prepared by reverse transcription reaction, the above primers (2 $\mu$M each), dNTP (0.2 mM each), and 0.65 U of ExTaq polymerase (Takara Shuzo)). As a result, as shown in Fig. 1, strong expression of the canine MRAP2 gene was observed in mastocytoma and perianal adenocarcinoma in the case of canine tumor tissues (Fig. 1). Furthermore, expression of the human MRAP2 gene was not observed in almost all healthy human tissues (Fig. 2A). On the other hand, strong expression of the human MRAP2 gene was observed in the cell lines of leukemia, malignant lymphoma, lung cancer, brain tumor, colorectal cancer, pancreatic cancer, gastric cancer, liver cancer, ovary cancer, esophageal cancer, and kidney cancer in the case of human cancer cells (Fig. 2B). Furthermore, expression of the mouse MRAP2 gene was detected in the cell lines of leukemia, melanoma, and neuroblastoma (Fig. 3).

Example: 2 Preparation of human MRAP2 protein

(1) Cloning of full-length cDNA encoding human MRAP2, and cDNA encoding extracellular region of human MRAP2 (hereinafter, referred to as hN-terminal portion of MRAP2 or hC-terminal portion of MRAP2, respectively)

Full-length cDNA encoding human MRAP2 was cloned by the following method based on the gene of SEQ ID NO: 1 obtained in Example 1. PCR was performed by repeating 30 times a cycle of 98°C/10 seconds, 55°C/15 seconds, and 72°C/1 minute using a Thermal Cycler (BIO RAD) and a reaction solution adjusted to a total amount of 50 $\mu$l through addition of each reagent and an attached buffer (1 $\mu$l of cDNA (which was from a variety of tissue/cell-derived cDNAs prepared in Example 1 and observed for their expression by RT-PCR), 2 types of primers (0.4 $\mu$M each; SEQ ID NOS: 33 and 34) containing *Eco*RI and *Not*I restriction enzyme cleavage sequences, 0.2 mM dNTP, 1.25 U PrimeSTAR HS polymerase (Takara Shuzo). The above 2 types of primers were used to amplify the region encoding the full-length amino acid sequence of SEQ ID NO: 2. After PCR, the thus amplified DNA was subjected to 1% agarose gel electrophoresis and then a DNA fragment of approximately 0.6 kbp was purified using a QIAquick Gel Extraction Kit (QIAGEN). The thus obtained PCR amplification product was inserted into pcDNA3.1 (Thermo Fisher Scientific) (hereinafter, the resultant is referred to as human MRAP2/pcDNA3.1). The amplification product was also confirmed, by sequencing using a DNA sequencer, to have a cDNA sequence encoding human MRAP2. The sequence shown in SEQ ID NO: 1 is the nucleotide sequence of the human MRAP2 gene, and the sequence shown in SEQ ID NO: 2 is the amino acid sequence of the human MRAP2 protein.

Further, PCR was performed based on SEQ ID NO: 1 by repeating 30 times a cycle of 98°C/10 seconds, 55°C/15 seconds, and 72°C/30 seconds using a Thermal Cycler (BIO RAD) and a reaction solution adjusted to a total amount of 50 $\mu$l through addition of each reagent and an attached buffer (2 types of primers (0.4 $\mu$M each; SEQ ID NOS: 35 and 36) containing *Kpn*I and *EcoR*I restriction enzyme cleavage sequences, 0.2 mM dNTP, 1.25 U PrimeSTAR HS polymerase (Takara Shuzo)). The above 2 types of primers were used to amplify the region encoding the amino acid sequence (SEQ ID NO: 10) of the extracellular region (N-terminal) of the MRAP2 protein, in SEQ ID NO: 1. After PCR, the thus amplified DNA was subjected to 1% agarose gel electrophoresis and then a DNA fragment of approximately 130 bp was purified using a QIAquick Gel Extraction Kit (QIAGEN). The thus obtained PCR amplification product was ligated to pSecTagB (Thermo Fisher Scientific) having an insert of cDNA encoding the mouse IgG2a Fc protein to prepare an expression vector encoding a human N-terminal MRAP2 extracellular region/mouse IgG2a Fc fusion protein (hereinafter, referred to as hN-terminal MRAP2-mIgG2aFc) (hereinafter, the obtained expression vector is referred to as pSecB-hN-terminal MRAP2-mIgG2aFc). The amplification product was also confirmed, by sequencing using a DNA sequencer, to have a cDNA sequence encoding hN-terminal MRAP2-mIgG2aFc. The sequence shown in SEQ ID NO: 37 is the nucleotide sequence encoding hN-terminal MRAP2-mIgG2aFc, and the sequence shown in SEQ ID NO: 38 is the amino acid sequence of hN-terminal MRAP2-mIgG2aFc.

Further, PCR was performed based on SEQ ID NO: 1 by repeating 30 times a cycle of 98°C/10 seconds, 55°C/15 seconds, and 72°C/30 seconds using a Thermal Cycler (BIO RAD) and a reaction solution adjusted to a total amount of 50 μl through addition of each reagent and an attached buffer (2 types of primers (0.4 μM each; SEQ ID NOS: 39 and 40) containing *Kpn*I and *Eco*RI restriction enzyme cleavage sequences, 0.2 mM dNTP, 1.25 U PrimeSTAR HS polymerase (Takara Shuzo)). The above 2 types of primers were used to amplify the region encoding the amino acid sequence (SEQ ID NO: 12) of the extracellular region (C-terminal) of the MRAP2 protein, in SEQ ID NO: 1. After PCR, the thus amplified DNA was subjected to 1% agarose gel electrophoresis and then a DNA fragment of approximately 400 bp was purified using a QIAquick Gel Extraction Kit (QIAGEN). The thus obtained PCR amplification product was ligated to pSecTagB (Thermo Fisher Scientific) having an insert of cDNA encoding the mouse IgG2a Fc protein to prepare an expression vector encoding a human C-terminal MRAP2 extracellular region/mouse IgG2a Fc fusion protein (hereinafter, referred to as hC-terminal MRAP2-mIgG2aFc) (hereinafter, the obtained expression vector is referred to as pSecB-hC-terminal MRAP2-mIgG2aFc). The amplification product was also confirmed, by sequencing using a DNA sequencer, to have a cDNA sequence encoding hC-terminal MRAP2-mIgG2aFc. The sequence shown in SEQ ID NO: 41 is the nucleotide sequence encoding hC-terminal MRAP2-mIgG2aFc, and the sequence shown in SEQ ID NO: 42 is the amino acid sequence of hC-terminal MRAP2-mIgG2aFc.

(2) Preparation of hN-terminal MRAP2-mIgG2aFc

hN-terminal MRAP2-mIgG2aFc was prepared as an immunizing antigen for preparing antibodies to MRAP2.

The expression vector pSecB-hN-terminal MRAP2-mIgG2aFc was introduced by the lipofection method into human embryonic kidney cell line HEK293 cells and purification of hN-terminal MRAP2-mIgG2aFc was carried out from a culture supernatant obtained 7 days after introduction. The culture supernatant was applied to a Hi Trap Protein A HP column (GE Healthcare Bio-Science), which was then washed with a binding buffer (20 mM sodium phosphate (pH 7.0)), followed by elution with an elution buffer (0.1 M glycine-HCl (pH 2.7)). Eluates were immediately neutralized by elution into a tube supplemented with a neutralization buffer (1 M Tris-HCl (pH 9.0)). Next, the buffer in the eluates obtained by the above method was replaced with physiological phosphate buffer (Nissui Pharmaceutical Co., Ltd.) using ultrafiltration NANOSEP 10K OMEGA (PALL). Sterilized filtration was performed using 0.22-μm HT Tuffryn Acrodisc (PALL) and then the resultants were used for the following experiments.

(3) Preparation of hC-terminal MRAP2-mIgG2aFc

hC-terminal MRAP2-mIgG2aFc was prepared as an immunizing antigen for preparing antibodies to MRAP2.

The expression vector pSecB-hC-terminal MRAP2-mIgG2aFc was introduced by the lipofection method into human embryonic kidney cell line HEK293 cells and purification of hC-terminal MRAP2-mIgG2aFc was carried out from a culture supernatant obtained 7 days after introduction. The culture supernatant was applied to a Hi Trap Protein A HP column (GE Healthcare Bio-Science), which was then washed with a binding buffer (20 mM sodium phosphate (pH 7.0)), followed by elution with an elution buffer (0.1 M glycine-HCl (pH 2.7)). Eluates were immediately neutralized by elution into a tube supplemented with a neutralization buffer (1 M Tris-HCl (pH 9.0)). Next, the buffer in the eluates obtained by the above method was replaced with physiological phosphate buffer (Nissui Pharmaceutical Co., Ltd.) using ultrafiltration NANOSEP 10K OMEGA (PALL). Sterilized filtration was performed using 0.22-μm HT Tuffryn Acrodisc (PALL) and then the resultants were used for the following experiments.

Example 3: Preparation of polyclonal antibody binding to extracellular region of MRAP2

(1) Preparation of polyclonal antibody to N-terminal portion of MRAP2

To obtain an antibody binding to the N-terminal extracellular region of MRAP2, hN-terminal MRAP2-mIgG2aFc (0.1 mg) prepared as described above as an antigen was mixed with a complete Freund's adjuvant (CFA) solution in an amount equivalent thereto. The mixture was subcutaneously administered to a mouse 4 times every 2 weeks. Subsequently, blood was collected, so that an antiserum containing a polyclonal antibody was obtained. Furthermore, the antiserum was purified using a protein G carrier (GE Healthcare BioSciences) and then a polyclonal antibody against hN-terminal MRAP2-mIgG2aFc was obtained. In addition, an antibody obtained by purifying serum of mice to which no antigen had been administered with the use of a protein G carrier in the manner described above was designated as a control antibody.

(2) Preparation of polyclonal antibody to C-terminal portion of MRAP2

To obtain an antibody binding to the C-terminal extracellular region of MRAP2, hC-terminal MRAP2-mIgG2aFc (0.1 mg) prepared as described above as an antigen was mixed with a complete Freund's adjuvant (CFA) solution in an amount equivalent thereto. The mixture was subcutaneously administered to a mouse 4 times every 2 weeks. Subsequently, blood was collected, so that an antiserum containing a polyclonal antibody was obtained. Furthermore, the antiserum was purified using a protein G carrier (GE Healthcare BioSciences) and then a polyclonal antibody against hC-terminal MRAP2-mIgG2aFc was obtained. In addition, an antibody obtained by purifying serum of mice to which no antigen had been administered with the use of a protein G carrier in the manner described above was designated as a control antibody.

(3) Establishment of cells stably expressing full-length human MRAP2

Human MRAP2/pcDNA3.1 prepared as described above was introduced by the lipofection method into CHO-K1 cells (ATCC) and then selection was performed using 500 μg/ml G418 (Nacalai Tesque, Inc.) to establish a CHO cell line stably expressing full-length human MRAP2 (CHO-human MRAP2). Cells obtained by introducing an expression vector (hereinafter, referred to as emp/pcDNA3.1) having no insert of cDNA encoding MRAP2 and then performing selection in the manner described above was designated as control cells (hereinafter, referred to as CHO-emp).

(4) Analysis of antigen protein expression on cell surface

It was examined whether or not the polyclonal antibody prepared in (1) above specifically reacted with MRAP2 expressed on the surfaces of the full-length human MRAP2-stably expressing cells established in (3) above. The CHO-human MRAP2 cells or the CHO-emp cells ($10^6$ cells each) were centrifuged in a 1.5-ml microcentrifugal tube. The polyclonal antibody against N-terminal portion of MRAP2 (2 μg in 5 μl) prepared in (1) above was added thereto. The resultant was further suspended in PBS containing 0.1% fetal bovine serum (95 μl) and then left to stand on ice for 1 hour. After washing with PBS, the resultant was suspended in PBS containing an FITC-labeled goat anti-mouse IgG antibody (Santa Cruz Biotechnology, Inc.) (5 μl) and 0.1% fetal bovine serum (FBS) (95 μl) and then left to stand on ice for 1 hour. After washing with PBS, fluorescence intensity was measured using FACSCalibur (Becton, Dickinson and Company). Meanwhile, a procedure similar to the above was performed using the control antibody prepared in (1) above instead of the polyclonal antibody against MRAP2, so that a control was prepared. As a result, 215% increase in fluorescence intensity was found in the CHO-human MRAP2 cells to which the anti-N-terminal portion of MRAP2 polyclonal antibody had been added, as compared with the control. Meanwhile, a procedure similar to the above was performed for the CHO-emp cells. As a result, 0% increase in fluorescence intensity was found in the CHO-emp cells to which the anti-N-terminal portion of MRAP2 polyclonal antibody had been added, as compared with the control. Based on the above, it was revealed that the anti-N-terminal portion of MRAP2 polyclonal antibody was capable of specifically binding to the MRAP2 protein expressed on the cell membrane surfaces. In addition, the rate of increase in fluorescence intensity is represented by the rate of increase in mean fluorescence intensity (MFI value) in cells. It was calculated by the following equation.

$$\text{Rate of increase in mean fluorescence intensity (rate of increase in fluorescence intensity) (\%)} = ((\text{MFI value of cells reacted with an anti-human MRAP2 antibody}) - (\text{control MFI value})) / (\text{control MFI value}) \times 100$$

Next, it was examined whether or not the polyclonal antibody prepared in (2) above specifically reacted with MRAP2 expressed on the surfaces of the full-length human MRAP2-stably expressing cells established in (3) above. The CHO-human MRAP2 cells or the CHO-emp cells ($10^6$ cells each) were centrifuged in a 1.5-ml microcentrifugal tube. The polyclonal antibody against C-terminal portion of MRAP2 (2 μg in 5 μl) prepared in (2) above was added thereto. The resultant was further suspended in PBS containing 0.1% fetal bovine serum (95 μl) and then left to stand on ice for 1 hour. After washing with PBS, the resultant was suspended in PBS containing an FITC-labeled goat anti-mouse IgG antibody (Santa Cruz Biotechnology, Inc.) (5 μl) and 0.1% fetal bovine serum (FBS) (95 μl) and then left to stand on ice for 1 hour. After washing with PBS, fluorescence intensity was measured using FACSCalibur (Becton, Dickinson and Company). Meanwhile, a procedure similar to the above was performed using the control antibody prepared in (2) above instead of the polyclonal antibody against MRAP2, so that a control was prepared. As a result, 223% increase in fluorescence intensity was found in the CHO-human MRAP2 cells to which the anti-C-terminal portion of MRAP2 polyclonal antibody had been added, as compared with the control. Meanwhile, a procedure similar to the above was performed for the CHO-emp cells. As a result, 0% increase in fluorescence intensity was found in the CHO-emp cells to which the anti-C-terminal portion of MRAP2 polyclonal antibody had been added, as compared with the control. Based on the above, it was revealed that the anti-C-terminal portion of MRAP2 polyclonal antibody was capable of specifically binding to the MRAP2 protein expressed on the cell membrane surfaces. In addition, the rate of increase in fluorescence intensity is represented by the rate of increase in mean fluorescence intensity (MFI value) in cells. It was calculated by the following equation.

$$\text{Rate of increase in mean fluorescence intensity (rate of increase in fluorescence intensity) (\%)} \\ = ((\text{MFI value of cells reacted with an anti-human MRAP2 antibody}) - (\text{control MFI value})) / \\ (\text{control MFI value}) \times 100$$

Next, it was examined whether or not the MRAP2 protein was expressed on cell surfaces of 2 types of leukemia cell lines (K562 and THP-1) and a malignant lymphoma cell line (Namalwa) in which MRAP2 gene expression had been strongly observed. Each human cell line ($10^6$ cells) in which gene expression had been observed as described above was centrifuged in a 1.5-ml microcentrifugal tube. The polyclonal antibody against C-terminal portion of MRAP2 (2 $\mu$g in 5 $\mu$l) prepared in (2) above was added thereto. The resultant was further suspended in PBS containing 0.1% fetal bovine serum (95 $\mu$l) and then left to stand on ice for 1 hour. After washing with PBS, the resultant was suspended in PBS containing an FITC-labeled goat anti-mouse IgG antibody (Santa Cruz Biotechnology, Inc.) (5 $\mu$l) and 0.1% fetal bovine serum (FBS) (95 $\mu$l) and then left to stand on ice for 1 hour. After washing with PBS, fluorescence intensity was measured using FACSCalibur (Becton, Dickinson and Company). Meanwhile, a procedure similar to the above was performed using the control antibody prepared in (2) above instead of the polyclonal antibody against C-terminal portion of MRAP2, so that a control was prepared. As a result, fluorescence intensity was found to be at least 30% stronger in all cells to which the anti-C-terminal portion of MRAP2 polyclonal antibody had been added than that in control cells. Specifically, the following increases in fluorescence intensity were observed: K562: 197%, THP-1: 123%, and Namalwa: 104%. Based on the above, it was revealed that the MRAP2 protein was expressed on the cell membrane surfaces of the above human cancer cell lines. In addition, the rate of increase in fluorescence intensity is represented by the rate of increase in mean fluorescence intensity (MFI value) in cells. It was calculated by the following equation.

$$\text{Rate of increase in mean fluorescence intensity (rate of increase in fluorescence intensity) (\%)} \\ = ((\text{MFI value of cells reacted with an anti-human MRAP2 antibody}) - (\text{control MFI value})) / \\ (\text{control MFI value}) \times 100$$

Example 4: Antitumor effects (ADCC activity) of polyclonal antibody against MRAP2 tocancer cells

Next, it was examined whether or not a polyclonal antibody against MRAP2 would be able to damage MRAP2-expressing tumor cells. Evaluation was carried out using the polyclonal antibody against human N-terminal portion of MRAP2 or human C-terminal portion of MRAP2 prepared in Example 3. A human leukemia cell line K562 or a human malignant lymphoma cell line Namalwa ($10^6$ cells), in which MRAP2 expression had been confirmed, were separately collected into a 50-ml centrifugal tube. Chromium 51 (100 $\mu$Ci) was added thereto, followed by incubation at 37°C for 2 hours. Thereafter, cells were washed 3 times with an RPMI1640 medium containing 10% fetal bovine serum and added to wells ($10^3$ cells per well) in 96-well V-bottom plates. The above each polyclonal antibody against human MRAP2 was added thereto (1 $\mu$g per well). Further, lymphocytes separated from mouse peripheral blood were added thereto ($2 \times 10^5$ cells per well), followed by culture under conditions of 37°C and 5% $CO_2$ for 4 hours. After culture, the level of chromium (Cr) 51 released from damaged tumor cells in each culture supernatant was determined. Then, the ADCC activity of the polyclonal antibody against human N-terminal portion of MRAP2 or human C-terminal portion of MRAP2 to cancer cells was calculated. As a result, ADCC activities against the K562 cells (15.7% and 16.3%, respectively) and the Namalwa cells (14.8% and 14.0%, respectively) were observed (see Fig. 4). Meanwhile, substantially no activity against each cell line was observed in a case in which a procedure similar to the above was performed using the control antibody prepared from peripheral blood of a mouse that had not been immunized with an antigen (Example 3) or in a case in which no antibody was added (see Fig. 4). Accordingly, it was revealed that MRAP2-expressing tumor cells can be damaged by inducing the ADCC activity with the use of an antibody against MRAP2.

In addition, for cytotoxic activity (ADCC activity) in Fig. 4, an antibody against MRAP2 used in the present invention, mouse lymphocytes, and $10^3$ cells of the above cell lines incorporating chromium 51 were mixed together and cultured for 4 hours, and then the level of chromium 51 released into the medium was determined as described above. The cytotoxic activity to the leukemia cell line was calculated by the following equation*.

*Equation: Cytotoxic activity (%) = [(the level of chromium 51 released from K562 to which an antibody against MRAP2 and mouse lymphocytes were added) / (the level of chromium 51 released from target cells to which 1 N hydrochloric acid was added)] $\times$ 100

Industrial Applicability

The antibodies of the present invention are useful for treatment and/or prevention of cancers.

All publications, patents, and patent applications cited herein are incorporated herein by reference in their entirety.

## Claims

1. A pharmaceutical composition for treatment and/or prevention of a cancer, which comprises, as an active ingredient, an antibody or fragment thereof having an immunological reactivity with an MRAP2 protein having the amino acid sequence shown in SEQ ID NO: 2, 4, 6, or 8 or an amino acid sequence having 80% or more sequence identity with the amino acid sequence, or with a fragment of the MRAP2 protein comprising 7 or more consecutive amino acids.

2. The pharmaceutical composition according to claim 1, which comprises, as an active ingredient, an antibody or fragment thereof having an immunological reactivity with a partial polypeptide of the MRAP2 protein, the partial polypeptide being a polypeptide consisting of 7 or more consecutive amino acids of the amino acid sequence shown in any one of the even numbered SEQ ID NOS: 10 to 24, or a polypeptide consisting of an amino acid sequence having 80% or more sequence identity with the amino acid sequence.

3. The pharmaceutical composition according to claim 1 or 2, wherein the cancer is a cancer expressing MRAP2 on a cell surface.

4. The pharmaceutical composition according to any one of claims 1 to 3, wherein the cancer is selected from the group consisting of leukemia, malignant lymphoma, lung cancer, brain tumor, colorectal cancer, melanoma, neuroblastoma, pancreatic cancer, gastric cancer, liver cancer, ovary cancer, esophageal cancer, kidney cancer, mastocytoma, and perianal adenocarcinoma.

5. The pharmaceutical composition according to any one of claims 1 to 4, wherein the antibody is a monoclonal or polyclonal antibody.

6. The pharmaceutical composition according to any one of claims 1 to 5, wherein the antibody is a human antibody, a humanized antibody, a chimeric antibody, a single chain antibody, or a multispecific antibody.

7. An antibody or fragment thereof having an immunological reactivity with an N-terminal partial polypeptide of an MRAP2 protein, the partial polypeptide being a polypeptide consisting of the amino acid sequence shown in SEQ ID NO: 10, 14, 18, or 22 or an amino acid sequence having 80% or more sequence identity with the amino acid sequence.

**8.** An antibody or fragment thereof having an immunological reactivity with a C-terminal partial polypeptide of an MRAP2 protein, the partial polypeptide being a polypeptide consisting of the amino acid sequence shown in SEQ ID NO: 12, 16, 20, or 24 or an amino acid sequence having 80% or more sequence identity with the amino acid sequence.

**9.** The antibody or fragment thereof according to claim 7 or 8, wherein the antibody is a human antibody, a humanized antibody, a chimeric antibody, a single chain antibody, or a multispecific antibody.

**10.** A pharmaceutical combination for treatment and/or prevention of a cancer, which comprises the pharmaceutical composition according to any one of claims 1 to 6 and a pharmaceutical composition comprising an antitumor agent.

**11.** A method for treating and/or preventing a cancer, which comprises administering, to a subject, an antibody or fragment thereof having an immunological reactivity with an MRAP2 protein having the amino acid sequence shown in SEQ ID NO: 2, 4, 6, or 8 or an amino acid sequence having 80% or more sequence identity with the amino acid sequence, or with a fragment of the MRAP2 protein comprising 7 or more consecutive amino acids.

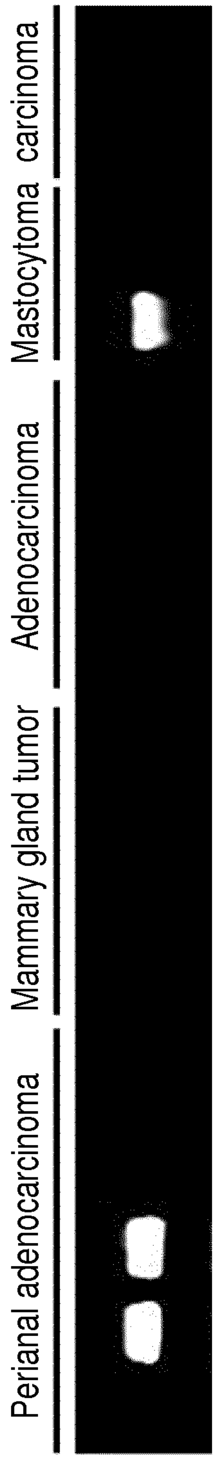
Perianal adenocarcinoma
Mammary gland tumor
Adenocarcinoma
Mastocytoma
Squamous cell carcinoma

Fig. 1

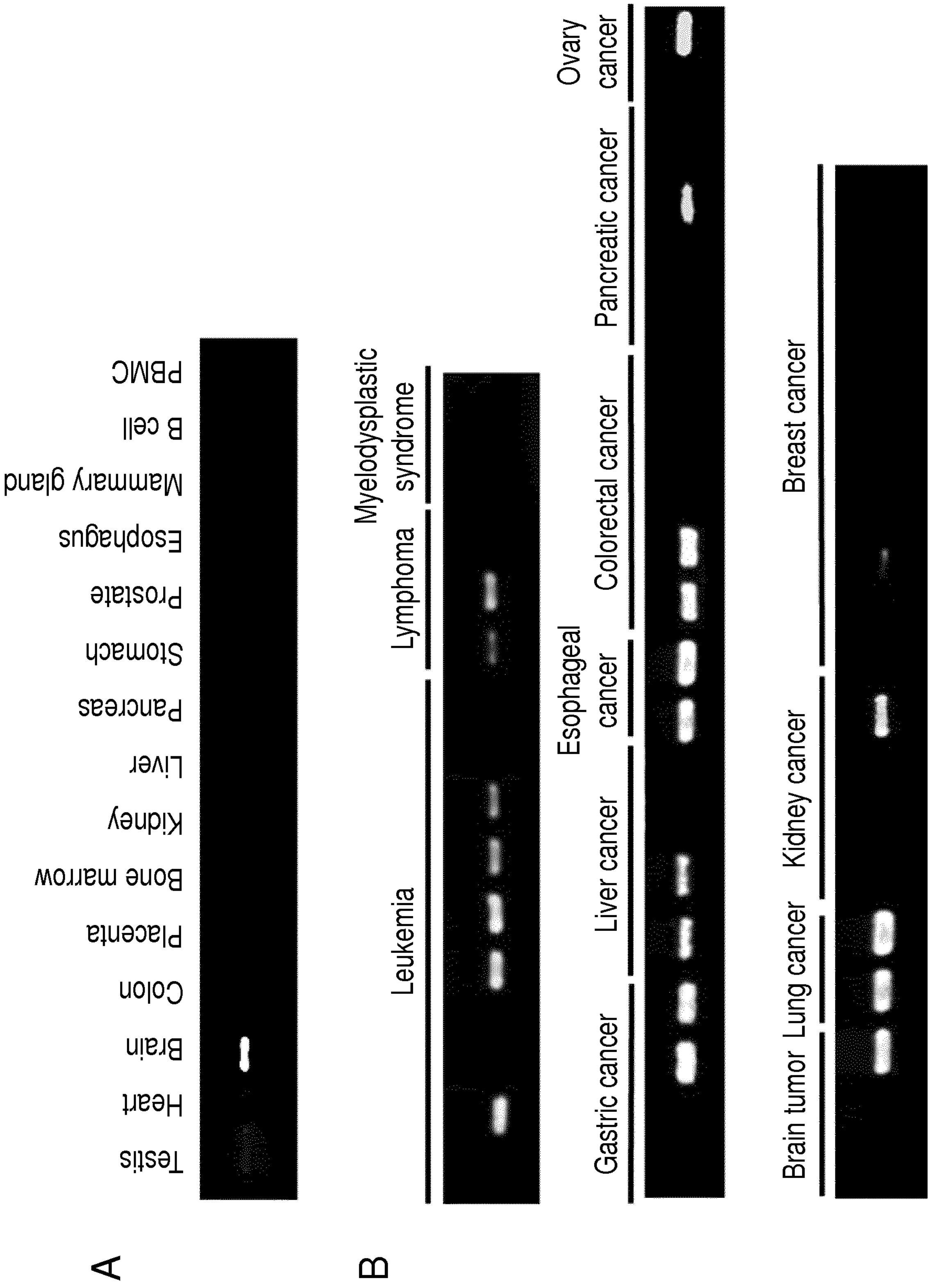
A
Testis
Heart
Brain
Colon
Placenta
Bone marrow
Kidney
Liver
Pancreas
Stomach
Prostate
Esophagus
Mammary gland
B cell
PBMC
B
Leukemia
Lymphoma
Myelodysplastic syndrome
Gastric cancer
Liver cancer
Esophageal cancer
Colorectal cancer
Pancreatic cancer
Ovary cancer
Brain tumor
Lung cancer
Kidney cancer
Breast cancer

Fig. 2

Fig. 3

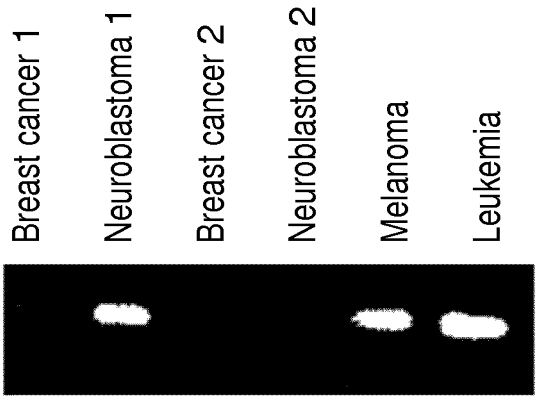
Breast cancer 1
Neuroblastoma 1
Breast cancer 2
Neuroblastoma 2
Melanoma
Leukemia

Fig. 4

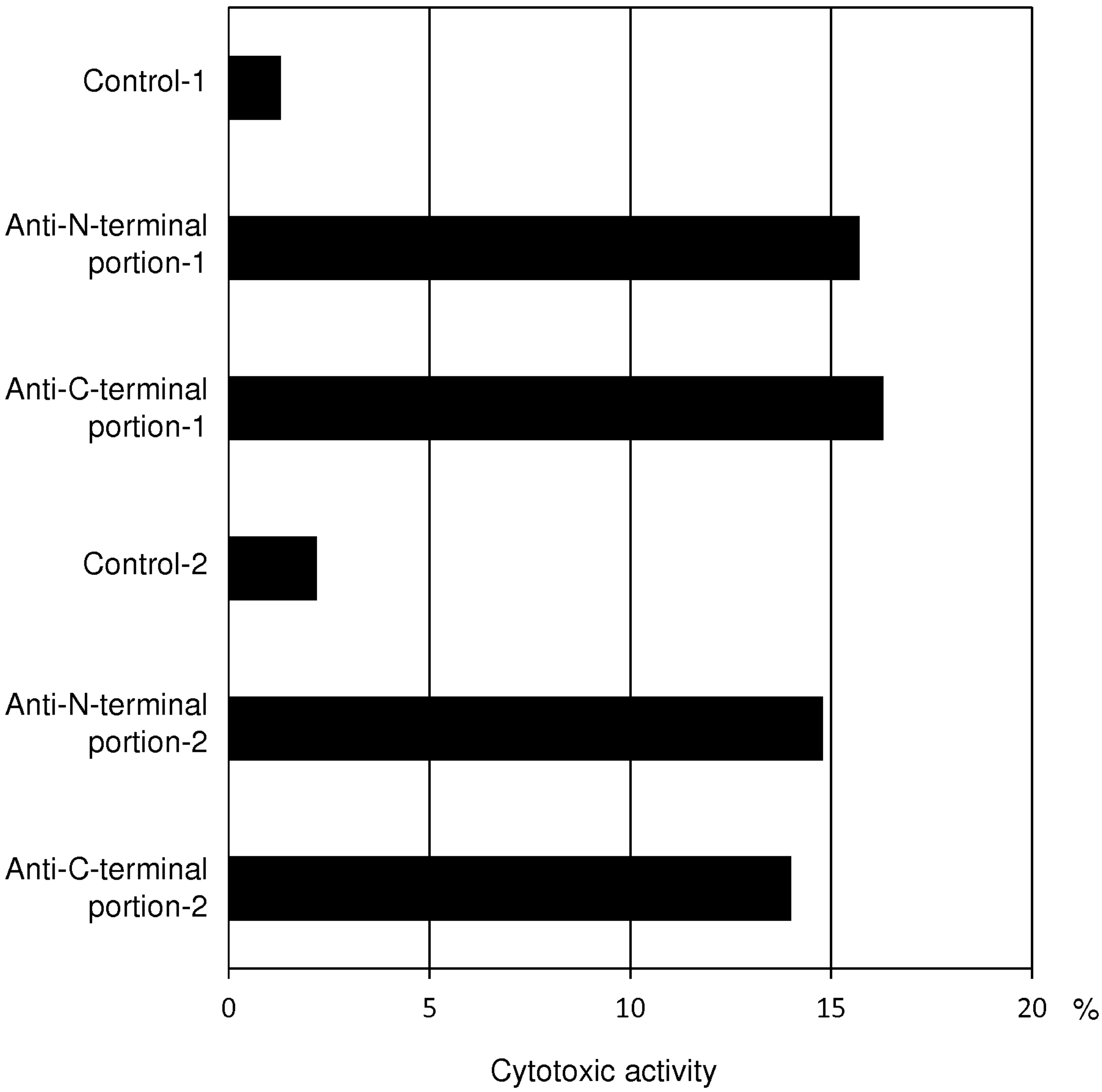
Control-1
Anti-N-terminal portion-1
Anti-C-terminal portion-1
Control-2
Anti-N-terminal portion-2
Anti-C-terminal portion-2
0
5
10
15
20
%
Cytotoxic activity

## INTERNATIONAL SEARCH REPORT

International application No. PCT/JP2017/012263

A. CLASSIFICATION OF SUBJECT MATTER
*A61K39/395*(2006.01)i, *A61K45/00*(2006.01)i, *A61P35/00*(2006.01)i, *A61P35/02*(2006.01)i, *C07K16/28*(2006.01)i, *C07K16/46*(2006.01)i, *C12N15/09*(2006.01)i, *C12P21/08*(2006.01)i

According to International Patent Classification (IPC) or to both national classification and IPC

B. FIELDS SEARCHED

Minimum documentation searched (classification system followed by classification symbols)
A61K39/395, A61K45/00, C07K16/28, C07K16/46, C12N15/09, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN), GenBank/EMBL/DDBJ/GeneSeq, UniProt

C. DOCUMENTS CONSIDERED TO BE RELEVANT

| Category* | Citation of document, with indication, where appropriate, of the relevant passages | Relevant to claim No. |
|---|---|---|
| X<br>A | WO 2015/006437 A1 (MAJZOUB, Joseph A.), 15 January 2015 (15.01.2015), paragraph [00141]<br>& JP 2016-523561 A & US 2016/0143256 A1<br>& EP 3019005 A1 & CA 2917961 A<br>& AU 2014287250 A & CN 105555132 A<br>& KR 10-2016-0042884 A | 7-8<br>1-6,9-11 |
| X<br>A | BANNON, MJ et al., "A Molecular Profile of Cocaine Abuse Includes the Differential Expression of Genes that Regulate Transcription, Chromatin, and Dopamine Cell Phenotype", Neuropsychopharmacol., 2014.08., Vol.39, No.9, p.2191-2199, ISSN 0893-133X, particularly, page 2193, column of 'Immunohistochemistry and Microscopy' | 7-8<br>1-6,9-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 April 2017 (28.04.17) | 23 May 2017 (23.05.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/012263

C (Continuation). DOCUMENTS CONSIDERED TO BE RELEVANT

| Category* | Citation of document, with indication, where appropriate, of the relevant passages | Relevant to claim No. |
|---|---|---|
| T | Product Datasheet "MRAP2 Antibody NBP1-88752" v.20.1 (Updated 10/23/2016), Earn rewards for product reviews and publications, p.1-3 [online], [retrieved on 2017.04.27], Retrieved from the Internet: <URL: http://www.novusbio.com/PDFs/NBP1-88752.pdf>, particularly, page 1, columns of 'Product Information', 'Product Description', page 2, column of 'Publications' | 7-8 |
| A | WO 2012/142330 A1 (BROWN UNIVERSITY), 18 October 2012 (18.10.2012), page 51, 9th line from the bottom to page 54, 5th line from the bottom (Family: none) | 1-6,9-11 |
| A | ASAI, M et al., "Loss of function of the melanocortin 2 receptor accessory protein 2 is associated with mammalian obesity", Science, 2013.07.19, Vol.341, No.6143, p.275-278, ISSN 0036-8075, particularly, Abstract | 1-6,9-11 |
| A | HWANG, GW et al., "siRNA-mediated knockdown of the melanocortin 2 receptor accessory protein 2 (MRAP2) gene confers resistance to methylmercury on HEK293 cells", J. Toxicol.Sci., 2010.12., Vol.35, No.6, p.947-950, ISSN 0388-1350, particularly, ABSTRACT | 1-6,9-11 |
| A | HAFIZ, S et al., "Expression of melanocortin receptors in human prostate cancer cell lines: MC2R activation by ACTH increases prostate cancer cell proliferation", Int. J. Oncol., 2012.10., Vol.41, No.4, p.1373-1380, ISSN 1019-6439, particularly, Abstract | 1-6,9-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016064033 A
- WO 0243478 A
- WO 02092812 A
- JP 2007530068 A
- WO 9846777 A
- EP 239400 A
- WO 9602576 A
- WO 9951743 A


### Non-patent literature cited in the description


- **JACKSON DS. et al.** *Front. Neurosci,* 2015, vol. 9, 213
- **ASAI M. et al.** *Science,* 2013, vol. 341, 275-278
- **KARLIN ; ALTSCHUL.** *Proc. Natl. Acad. Sci. USA,* 1993, vol. 90, 5873-5877
- **ALTSCHUL et al.** *Nucleic Acids Res.,* 1997, vol. 25, 3389-3402
- Protein Chemistry IV, Chemical Modification and Peptide Synthesis. Biochemical Experimentation Course (Seikagaku Jikken Koza) 1. Kagaku-dojin Publishing Company, Inc, 1981
- Current Protocols in Molecular Biology. **SAMBROOK et al.** Molecular Cloning. Cold Spring Harbor Laboratory Press, 1989
- A Compendium of Methods from Current Protocols in Molecular Biology. **AUSUBEL et al.** Short Protocols in Molecular Biology. John Wiley & Sons, 1995
- Current Protocols in Molecular Biology. **SAMBROOK et al.** Molecular Cloning. 1989
- *J. Immunol.,* 1979, vol. 123, 1548-1550
- *Current Topics in Microbiology and Immunology,* 1978, vol. 81, 1-7
- **KOHLER. G. ; MILSTEIN, C.** *Eur. J. Immunol.,* 1976, vol. 6, 511-519
- **MARGULIES. D. H. et al.** *Cell,* 1976, vol. 8, 405-415
- **SHULMAN, M. et al.** *Nature,* 1978, vol. 276, 269-270
- **DE ST. GROTH, S. F. et al.** *J. Immunol. Methods,* 1980, vol. 35, 1-21
- **TROWBRIDGE, I. S.** *J. Exp. Med.,* 1978, vol. 148, 313-323
- **GALFRE, G. et al.** *Nature,* 1979, vol. 277, 131-133
- **KOHLER, G. ; MILSTEIN, C.** *Methods Enzymol.,* 1981, vol. 73, 3-46
- **CARL, A. K. BORREBAECK ; JAMES, W. LARRICK.** THERAPEUTIC MONOCLONAL ANTIBODIES. MACMILLAN PUBLISHERS LTD, 1990
- **SATO K. et al.** *Cancer Research,* 1993, vol. 53, 851-856
- **HASHIMOTO-GOTOH, T. et al.** *Gene,* 1995, vol. 152, 271-275
- **ZOLLER, MJ. ; SMITH, M.** *Methods Enzymol.,* 1983, vol. 100, 468-500
- **KRAMER, W. et al.** *Nucleic Acids Res.,* 1984, vol. 12, 9441-9456
- **KRAMER, W. ; FRITZ, HJ.** *Methods Enzymol.,* 1987, vol. 154, 350-367
- **KUNKEL, TA.** *Proc. Natl. Acad. Sci. USA.,* 1985, vol. 82, 488-492
- **KUNKEL.** *Methods Enzymol.,* 1988, vol. 85, 2763-2766
- **KABAT et al.** Sequences of Proteins of Immunological Interest. National Institute of Health, 1991
- **SAMBROOK et al.** Molecular Cloning A Laboratory Manual. Cold Spring Harbor Laboratory Press, 1989
- **G. -B. KIM et al.** *Protein Engineering Design and Selection,* 2007, vol. 20 (9), 425-432
- **P. J. DELVES.** ANTIBODY PRODUCTION ESSENTIAL TECHNIQUES. WILEY, 1997
- **P. SHEPHERD ; C. DEAN.** Monoclonal Antibodies. OXFORD UNIVERSITY PRESS, 2000
- **J. W. GODING.** Monoclonal Antibodies: Principles and Practice. ACADEMIC PRESS, 1993